# EUROPÄISCHE PATENTSCHRIFT

(11) **EP 3 523 271 B1**
(45) Veröffentlichungstag und Bekanntmachung des Hinweises auf die Patenterteilung: **09.12.2020**
(21) Anmeldenummer: 17778316.4
(22) Anmeldetag: 10.10.2017
(51) Int. Cl.: C07C 209/36, C07C 211/51

(54) **KATALYSATORMODIFIKATION MIT ALKALIMETALL-, ERDALKALIMETALL- ODER SELTENERDMETALLIONEN IN DER KONTINUIERLICHEN FLÜSSIGPHASEN-HYDRIERUNG VON NITROVERBINDUNGEN**
CATALYST MODIFICATION WITH ALKALINE METAL, ALKALINE EARTH METAL OR RARE EARTH METAL IONS IN CONTINUOUS LIQUID PHASE HYDRATION OF NITRO COMPOUNDS
MODIFICATION CATALYTIQUE AU MOYEN D'IONS DE MÉTAL DE TERRES RARES, DE MÉTAL ALCALINO-TERREUX OU DE MÉTAL ALCALIN DANS L'HYDROGÉNATION EN PHASE LIQUIDE CONTINUE DE COMPOSÉS NITRÉS

(30) Priorität: 10.10.2016 EP 16193137
(43) Veröffentlichungstag der Anmeldung: 14.08.2019
(73) Patentinhaber: BASF SE, 67056 Ludwigshafen am Rhein (DE)
(72) Erfinder: LANGE DE OLIVEIRA, Armin, 69123 Heidelberg (DE); WUCHER, Barbara, 67056 Ludwigshafen (DE); BECHTOLD, Christian, 67056 Ludwigshafen (DE); FRIKO, Michael, 67056 Ludwigshafen (DE); HEMPEL, Renate, 01945 Ruhland (DE)
(86) Internationale Anmeldenummer: PCT/EP2017/075746
(87) Internationale Veröffentlichungsnummer: WO 2018/069278

(56) Entgegenhaltungen:
- WO-A1-2005/092340
- WO-A2-2014/022116
- US-A1- 2006 014 761
- US-A1- 2013 190 500
- TELKAR M M ET AL: "Role of a co-metal in bimetallic Ni-Pt catalyst for hydrogenation of m-dinitrobenzene to m-phenylenediamine", APPLIED CATALYSIS A: GENERAL, ELSEVIER, AMSTERDAM, NL, Bd. 295, Nr. 1, 13. Oktober 2005 (2005-10-13), Seiten 23-30, XP027814501, ISSN: 0926-860X [gefunden am 2005-10-13]
- ZHENGKUN YU ET AL: "Hydrogenation of nitroaromatics by polymer-anchored bimetallic palladium-ruthenium and palladium-platinum catalysts under mild conditions", JOURNAL OF MOLECULAR CATALYSIS A: CHEMICAL., Bd. 120, Nr. 1-3, 1. Juni 1997 (1997-06-01), Seiten 247-255, XP055356085, NL ISSN: 1381-1169, DOI: 10.1016/S1381-1169(96)00420-7
- MANE, R.B. ET AL: "Selectivity Tuning Options in Hadrogenation of m-Chloronitrobenzene to m-Chloroaniline over Mono- and Bimetallic Supported Catalysts", INDUSTRIAL AND ENGINEERING CHEMISTRY RESEARCH, Bd. 51, 2012, Seiten 15564-15572, XP002768353,

## Beschreibung

Die vorliegende Erfindung betrifft ein Verfahren zur kontinuierlichen Hydrierung einer Nitroverbindung zu dem entsprechenden Amin in einem die Nitroverbindung enthaltenden flüssigen Reaktionsgemisch in Gegenwart eines Trägerkatalysators, der als aktive Komponente mindestens ein Element aus den Gruppen 7 bis 12 des Periodensystems der Elemente enthält, dadurch gekennzeichnet, dass die Hydrierung in Gegenwart mindestens eines Salzes der Alkalimetalle, Erdalkalimetalle oder der Seltenerdmetalle durchgeführt wird.

Verfahren zur kontinuierlichen Hydrierung von Nitroverbindungen zu den entsprechenden Aminen sind an sich bekannt.

CN 104402731 A beschreibt ein Verfahren zur Herstellung von Anilinverbindungen durch katalytische Hydrierung von Mononitrobenzolverbindungen. Die Reaktion wird im diskontinuierlichen Verfahren bei einer Temperatur von 20 bis 80°C durchgeführt. Der Reaktion werden 1 bis 10 mol-% Metallsalze, bezogen auf die Gesamtmenge des Substrates, zugeführt. Nachteilig bei dem in CN 104402731 A beschriebenen Verfahren ist, dass es diskontinuierlich durchgeführt wird, was zu unnötigen Totzeiten des Reaktors, erhöhtem Katalysatorverbrauch und deutlichen Qualitätsschwankungen zwischen den unterschiedlichen Chargen führt. Ein weiterer Nachteil liegt darin, dass große Mengen an Metallsalzen zugeführt werden müssen, was erhöhte Produktionskosten verursacht. Des Weiteren fördern die verwendeten Salze (Halogenide) die Metallkorrosion.

Darüber hinaus ist eine häufig beschriebene Schwierigkeit bei Verfahren zur Hydrierung von Nitroverbindungen zu den entsprechenden Aminen die Freisetzung großer Mengen an Reaktionswärme und damit verbundene potenziell hohe Reaktionstemperaturen.

So beschreibt DE 10 2008 063308 B4 ein Verfahren zur Herstellung von Toluylendiamin durch Hydrierung von Dinitrotoluol, bei dem die Reaktionswärme zur Gewinnung von Dampf genutzt wird, welcher in ein Dampfnetz einer industriellen Anlage eingespeist und weiter genutzt werden kann. Zur Dampferzeugung sind zwingend Reaktionstemperaturen größer 100°C erforderlich. Die in DE 10 2008 063308 B4 offenbarte Hydrierung wird bei einer Temperatur größer oder gleich 180°C durchgeführt.

Ein häufig beschriebenes Problem hoher Reaktionstemperaturen während einer Hydrierung sind jedoch unerwünschte Nebenreaktionen, welche beispielsweise auch durch örtliche Überhitzungen innerhalb des Reaktors ablaufen.

WO 2014/108351 A1 beschreibt eine Vorrichtung des Typs Loop-Venturi-Reaktor für die kontinuierliche Umsetzung von Flüssigkeiten mit Gasen, insbesondere für Hydrierungen, Oxidationen oder Acetylierungen, z.B. für die Herstellung von Toluylendiamin durch Hydrierung von Dinitrotoluol. Lokale Temperaturspitzen bei großer freiwerdender Reaktionswärme werden durch Modifizierung der Anordnung der Wärmeübertragerrohre des Reaktors vermieden. Als Katalysatoren werden beispielsweise aktivierte Nickelkatalysatoren gemäß WO 2008/145179 A1 verwendet, welche aus einer dotierten Ni/AI-Legierung bestehen und nicht geträgert sind. Ein Problem solcher Ni/AI-Legierungen ist die Entstehung von Nickelaluminaten, wie Takovit oder Takovit ähnlichen Verbindungen. Nickelaluminate entstehen bei der Hydrierung von Nitroaromaten, wenn der Ni/Al-Katalysator mehr als 5,5 Gew.-% Al enthält. Sie bilden Feststoffe, welche sich an den Wänden des Reaktors und den peripheren Apparaturen, wie Pumpen, festsetzen. Dies kann zur Verringerung der Effizienz der Wärmeübertragung des Systems und sogar zu Verstopfungen des Systems führen. Zur Dotierung der Ni/Al-Legierung des aktivierten Nickelkatalysators aus WO 2008/145179 A1 werden eines oder mehrere Metalle, ausgewählt aus der Gruppe bestehend aus Mg, Ce, Ti, V, Nb, Cr, W, Mn, Re, Fe, Ru, Co, Rh, Ir, Pt, Cu, Ag, Au und Bi, verwendet, um die Bildung von Nickelaluminaten, wie Takovit oder Takovit ähnlichen Verbindungen, zu reduzieren oder ganz zu vermeiden.

WO 00/35852 offenbart ein Verfahren zur Herstellung von Aminen durch katalytische Hydrierung von Nitroverbindungen. Dem Problem des verstärkten Ablaufs von unerwünschten Nebenreaktionen wird durch Optimierung der Bau- und Fahrweise des verwendeten Reaktors begegnet, welche die für die Nebenreaktionen ursächlichen örtlichen Überhitzungen vermeiden sollen.

EP 1678118 B1 betrifft ein Verfahren zur Herstellung von Aminen durch katalytische Hydrierung der entsprechenden Nitroverbindungen. Um Nebenreaktionen, welche zur Bildung von hochmolekularen Nebenprodukten oder zur Bildung von Leichtsiedern führen, zurückzudrängen, wird die Selektivität des Verfahrens mithilfe eines Katalysators bestehend aus Platin und Nickel verbessert.

DE 10 2005 041532 A1 beschreibt ebenfalls ein Verfahren zur Herstellung von Aminen durch katalytische Hydrierung der entsprechenden Nitroverbindungen mit verminderten Nebenreaktionen und verbesserter Selektivität des Verfahrens unter Verwendung eines Katalysators bestehend aus Platin, Nickel und einem zusätzlichen Metall.

R.B. Mane et al.: "Selectivity Tuning Options in Hydrogenation of m-Chloronitrobenzene to m-Chloroaniline over Mono- and Bimetallic Supported Pt Catalysts", Industrial & Engineering Chemistry Research 2012, 51, 15564-15572, beschreiben die Hydrierung von m-Chlornitrobenzol zu *m*-Chloranilin an geträgerten Ni-, Pt und Ni/Pt-Katalysatoren in Gegenwart von Na₂CO₃. Im Falle der Hydrierung von *m*-Chlornitrobenzol zu *m*-Chloranilin an einem monometallischen Pt/C Katalysator wird in Gegenwart von Natriumcarbonat eine Selektivitätssteigerung von 84 % auf 94 % festgestellt. Dies wird auf die Unterdrückung der Dehydrohalogenierung von *m*-Chlornitrobenzol und *m*-Chloranilin zurückgeführt.

Ein weiteres Problem bei den bekannten Verfahren besteht darin, dass sich nach längerer Laufzeit des Reaktionsprozesses aufgrund der Alterung des Katalysators die Produktausbeute verringert. Beispielsweise können Verunreinigungen im zugeführten Edukt oder die Produkte unerwünschter Nebenreaktionen zur Alterung des Katalysators beitragen. Die Alterung des Katalysators kann zudem beschleunigt werden durch eine Unterbrechung in der Zufuhr des Edukts, beispielsweise während einer Betriebspause.

Bei Verwendung eines Reaktors mit Stoffrückführung, einem sogenannten Schlaufenreaktor, kann es Bereiche geben, in denen eine vollständige Umwandlung des Edukts stattfindet. In diesen Bereichen kann die Alterung des Katalysators beschleunigt sein, da durch die vollständige Umwandlung des Edukts die Bildung hochsiedender Komponenten, wie verschieden amino- und methylsubstituierten Diphenylamine, Hydrazodiphenylen und Phenazinen, vermehrt stattfindet.

Weiterhin führt eine Unterbrechung der Zufuhr von Nitroverbindungen bei gleichzeitiger Aufrechterhaltung der Reaktionsbedingungen (wie Temperatur, Druck, Wasserstoffstrom und Strömung im Schlaufenreaktor (Zirkulationsstrom)) sowie aller anderen Bedingungen zur Bildung hochsiedender Komponenten, wie verschiedenen amino- und methylsubstituierten Diphenylaminen, Hydrazodiphenylen und Phenazinen, welche den Katalysator deaktivieren können. Wird das Verfahren durch erneute Zufuhr von Nitroverbindungen fortgeführt, ist die Ausbeute an Aminen deutlich geringer und erholt sich im Allgemeinen nur langsam und unvollständig.

Aufgabe der vorliegenden Erfindung ist es, in einem Verfahren zur kontinuierlichen Hydrierung von Nitroverbindungen zu den entsprechenden Aminen die Aktivität des Katalysators längerfristig zu erhalten, ohne die Zufuhrrate von Edukten oder die Temperatur reduzieren zu müssen. Weiterhin ist es eine Aufgabe der vorliegenden Erfindung, die Bildung hochsiedender Komponenten, die zur Verunreinigung des Katalysators sowie zu dessen Deaktivierung führen, zu verringern.

Die Aufgabe wird gelöst durch ein Verfahren zur kontinuierlichen Hydrierung einer Nitroverbindung zu dem entsprechenden Amin in einem die Nitroverbindung enthaltenden flüssigen Reaktionsgemisch in Gegenwart eines Trägerkatalysators, der als aktive Komponente mindestens ein Element aus den Gruppen 7 bis 12 des Periodensystems der Elemente enthält, dadurch gekennzeichnet, dass die Hydrierung in Gegenwart mindestens eines Salzes der Alkalimetalle, Erdalkalimetalle oder der Seltenerdmetalle durchgeführt wird.

Die Aufgabe wird ebenfalls gelöst durch einen Trägerkatalysator zur kontinuierlichen Hydrierung einer Nitroverbindung zu dem entsprechenden Amin in einem die Nitroverbindung enthaltenden flüssigen Reaktionsgemisch, der als aktive Komponente mindestens ein Element aus den Gruppen 7 bis 12 des Periodensystems der Elemente enthält, dadurch gekennzeichnet, dass er mindestens ein Salz der Alkalimetalle, Erdalkalimetalle oder der Seltenerdmetalle enthält.

Es wurde überraschend gefunden, dass die Hydrierung in Gegenwart mindestens eines Salzes der Alkalimetalle, Erdalkalimetalle oder der Seltenerdmetalle die Produktausbeute an Aminen signifikant erhöht. Gleichzeitig wird die Bildung hochsiedender Komponenten während des Verfahrens verringert und die Lebenszeit des Katalysators gesteigert.

Überraschenderweise verringert sich auch die Bildung hochsiedender Komponenten, wenn sich vor einer Unterbrechung der Zufuhr von Nitroverbindungen Salze der Alkali-, Erdalkali- oder der Seltenerdmetalle oder Gemische daraus in dem Reaktor befinden. Die Reaktionsausbeute an Aminen erreicht unmittelbar nach Weiterführung der Reaktion durch Zufuhr von Nitroverbindungen wieder ihr ursprüngliches Niveau.

Im Allgemeinen enthält die aktive Komponente des Katalysators mindestens ein Element der Gruppe bestehend aus Nickel, Platin, Palladium, Eisen und Kobalt.

In einer ersten bevorzugten Ausführungsform enthält die aktive Komponente des Trägerkatalysators Nickel in Form von Nickelkristalliten mit einer bimodalen Nickelkristallitgrößenverteilung und weist einen Nickel-Gehalt von 60 bis 80 Gew.-%, bezogen auf die Gesamtmasse des Katalysators, sowie einen Reduktionsgrad von mindestens 70% auf.

In einer zweiten bevorzugten Ausführungsform enthält die aktive Komponente des Trägerkatalysators ein Gemisch aus Nickel und Platin sowie gegebenenfalls mindestens einem zusätzlichen Metall. Vorzugsweise enthält der Hydrierkatalysator dieser zweiten bevorzugten Ausführungsform 1 bis 5 Gew.-% Platin, 0,3 bis 1,5 Gew.-% Nickel, 0,05 bis 1,5 Gew.-% des mindestens einen zusätzlichen Metalls, sowie 94,65 bis 97,45 Gew.-% Trägermaterial, bezogen auf das Gesamtgewicht des Katalysators. Vorzugsweise ist das mindestens eine zusätzliche Metall Chrom.

Das mindestens eine Salz der Alkalimetalle, Erdalkalimetalle oder der Seltenerdmetalle kann beispielsweise im Katalysator enthalten sein, vorzugsweise insgesamt in einer Konzentration von 0,05 bis 20 Gew.-%, bezogen auf die Trockenmasse des Katalysators.

Ebenso kann das mindestens eine Salz der Alkalimetalle, Erdalkalimetalle oder der Seltenerdmetalle im flüssigen Reaktionsgemisch enthalten sein, vorzugweise insgesamt in einer Konzentration von 0,01 bis 1 mol-%, bezogen auf die zugeführte Menge an zu hydrierender Nitroverbindung.

Im Allgemeinen enthält das mindestens eine Salz der Alkalimetalle, Erdalkalimetalle oder der Seltenerdmetalle Kalium, Strontium, Natrium oder Gemische daraus und ist ein Carbonat, Hydrogencarbonat, Hydroxid, Oxid, Nitrat oder Carboxylat.

### Nitroverbindungen

In dem erfindungsgemäßen Verfahren werden als Nitroverbindung organische Verbindungen mit mindestens einer Nitrogruppe eingesetzt.

Geeignete Nitroverbindungen sind beispielsweise Nitroalkohole und Nitroaromaten.

Geeignete Nitroalkohole sind beispielsweise Tris(hydroxymethyl)nitromethan, 2-Nitro-2-methyl-1,3-propandiol, 2-Nitro-2-ethyl-1,3-propandiol, 2-Nitro-1-butanol und 2-Nitro-2-methyl-1-propanol sowie beliebige Gemische aus zwei oder mehr der genannten Nitroalkohole. Diese werden zu den entsprechenden Aminoalkoholen hydriert.

In einer bevorzugten Ausführungsform handelt es sich bei der zu hydrierenden Nitroverbindung um einen Nitroaromaten. Geeignete Nitroaromaten sind Mononitroaromaten, Dinitroaromaten und Polynitroaromaten. Bei Polynitroaromaten im Sinne der Erfindung handelt es sich um Nitroaromaten mit mindestens drei Nitrogruppen. Bevorzugt werden Mononitroaromaten oder Dinitroaromaten, besonders bevorzugt Dinitroaromaten hydriert.

Im Allgemeinen weisen die verwendeten Mononitroaromaten 6 bis 18 Kohlenstoffatome auf. Geeignete Mononitroaromaten sind Mononitrotoluole und deren Halogenderivate sowie Mononitrobenzole und deren Halogenderivate, zum Beispiel Nitrobenzol, o-Nitrotoluol, m-Nitrotoluol, p-Nitrotoluol, Mononitroxylole, wie 1,2-Dimethyl-3-nitrobenzol, 1,2-Dimethyl-4-nitrobenzol, 1,4-Dimethyl-2-nitrobenzol, 1,3-Dimethyl-2-nitrobenzol, 2,4-Dimethyl-1-nitrobenzol und 1,3-Dimethyl-5-nitrobenzol, Mononitronaphthaline, wie 1-Nitronaphthalin und 2-Nitronaphthalin, Chlornitrobenzole, wie o-Chlornitrobenzol,m-Chlornitrobenzol, p-Chlornitrobenzol, oder 1,2-Dichlor-4-nitrobenzol, 1,4-Dichlor-2-nitrobenzol, 2,4-Dichlor-1-nitrobenzol und 1,2-Dichlor-3-nitrobenzol, Chlornitrotoluole, wie 4-Chlor-2-nitrotoluol, 4-Chlor-3-nitrotoluol, 2-Chlor-4-nitrotoluol und 2-Chlor-6-nitrotoluol, und Nitroaniline, wie o-Nitroanilin, m-Nitroanilin oder p-Nitroanilin.

Bevorzugte Mononitroaromaten sind Mononitrobenzole, halogenierte Mononitrobenzole und Mononitrotoluole, besonders bevorzugt sind Nitrobenzol, o-Nitrotoluol, m-Nitrotoluol, p-Nitrotoluol, 1,2-Dimethyl-3-nitrobenzol, 1,2-Dimethyl-4-nitrobenzol, 1,4-Dimethyl-2-nitrobenzol, 1,3-Dimethyl-2-nitrobenzol, 2,4-Dimethyl-1-nitrobenzol, 1,2-Dichlor-4-nitrobenzol, 1,4-Dichlor-2-nitrobenzol, 2,4-Dichlor-1-nitrobenzol oder 1,2-Dichlor-3-nitrobenzol, ganz besonders bevorzugt sind o-Nitrotoluol, m-Nitrotoluol und p-Nitrotoluol.

Im Allgemeinen weisen die verwendeten Dinitroaromaten 6 bis 18 Kohlenstoffatome auf. Geeignete Dinitroaromaten sind Dinitrotoluole und deren Halogenide, Dinitrobenzole und deren Halogenide sowie Dinitronaphthaline. Beispielsweise können 1,3-Dinitrobenzol, 2,4-Dinitrotoluol, 2,6-Dinitrotoluol, Dinitroxylole, wie 2,4-Dinitro-m-xylol, 3,5-Dinitro-o-xylol, 4,5-Dinitro-o-xylol oder 4,6-Dinitro-m-xylol, Dinitronaphthaline, wie 1,5-Dinitronaphthalin oder 1,8-Dinitronaphthalin, oder Chlornitrobenzole, wie 2-Chlor-1,3-dinitrobenzol oder 1-Chlor-2,4-dinitrobenzol, verwendet werden.

Bevorzugte Dinitroaromaten sind Dinitrobenzole, halogenierte Dinitrobenzole, Dinitronaphthaline und Dinitrotoluole, besonders bevorzugt m-Dinitrobenzol, 1,5-Dinitronaphthalin, 1,3-Dinitrobenzol, 2,4-Dinitrotoluol, 2,6-Dinitrotoluol, 2-Chlor-1,3-dinitrobenzol und 1-Chlor-2,4-dinitrobenzol und ganz besonders bevorzugt m-Dinitrobenzol, 1,5-Dinitronaphthalin, 2,4-Dinitrotoluol und 2,6-Dinitrotoluol.

Im Allgemeinen weisen die verwendeten Polynitroaromaten 6 bis 18 Kohlenstoffatome auf. Geeignete Polynitroaromaten sind beispielweise 2,4,6-Trinitrotoluol, Polynitroxylole, wie 2,4,6-Trinitro-m-xylol oder 4,5,6-Trinitro-m-xylol.

Bevorzugte Polynitroaromaten sind 2,4,6-Trinitrotoluol, 2,4,6-Trinitro-m-xylol oder 4,5,6-Trinitro-m-xylol, besonders bevorzugt ist 2,4,6,-Trinitrotoluol.

Auch Mischungen der genannten Mononitroaromaten, Dinitroaromaten und Polynitroaromaten sind erfindungsgemäß geeignet.

Vorzugsweise handelt es sich bei den Nitroverbindungen um Nitroaromaten, bevorzugt um Dinitroaromaten, besonders bevorzugt um Dinitrobenzol, halogenierte Dinitrobenzole und Dinitrotoluole und ganz besonders bevorzugt um Dinitrotoluol.

In einer ganz besonders bevorzugten Ausführungsform wird 2,4-Dinitrotoluol oder 2,6-Dinitrotoluol eingesetzt. Auch technische Gemische enthaltend 2,4-Dinitrotoluol und 2,6-Dinitrotoluol sind geeignet, wobei diese Gemische vorzugsweise bis zu 35 Gew.-% an 2,6-Dinitrotoluol mit Anteilen von vorzugsweise 1 bis 5 Gew.-%, bevorzugt 1 bis 4 Gew.-% an vicinalem Dinitrotoluol und vorzugsweise 0,5 bis 1,5 Gew.-% an 2,5- und 3,5-Dinitrotoluol, bezogen auf das Gesamtgemisch, aufweisen.

Auch Gemische der genannten Nitroalkohole und Nitroaromaten sind erfindungsgemäß geeignet.

Die genannten Nitroalkohole und Nitroaromaten sind kommerziell erhältlich.

Weiterhin können die verwendeten Nitroalkohole und Nitroaromaten durch chemische Synthese gewonnen werden, wie beispielsweise Dinitrotoluole durch die Nitrierung von Toluol erhalten werden können. Das dabei entstehende Reaktionsprodukt enthält zumeist neben der gewünschten Nitroverbindung zahlreiche Verunreinigungen. Beispielsweise kann Nitriersäure umfassend Salpetersäure, Schwefelsäure und Stickstoffoxide in diesem Reaktionsprodukt enthalten sein. Auch Abbauprodukte, wie beispielsweise Distickstoffmonoxid, Cyanwasserstoffsäure, Kohlenstoffmonoxid oder Gemische daraus, können als Verunreinigungen enthalten sein. Ebenso können Oxidationsprodukte, beispielsweise aus unerwünschten Nebenreaktionen der Nitroaromaten, enthalten sein, wie z.B. aromatische Carbonsäuren, wie Nitrobenzoesäuren, beispielsweise Mononitrobenzoesäure, Dinitrobenzoesäure oder deren Abbauprodukte sowie Gemische daraus.

Weitere Verunreinigungen können in Form von Hochsiedern, wie Nitrokresol, beispielsweise Mononitrokresol, Dinitrokresol oder Trinitrokresol, und Nitrophenol, beispielsweise Dinitrophenol oder Trinitrophenol, vorliegen.

Im Allgemeinen ist daher eine Aufreinigung der so erhaltenen Nitroverbindungen notwendig, damit sie als Edukte für nachfolgende Verfahren, wie beispielsweise die Hydrierung zu den entsprechenden Aminen, geeignet sind. Verfahren zur Synthese von Nitroverbindungen sowie deren weitere Aufreinigung sind dem Fachmann allgemein bekannt oder beispielsweise für Dinitrotoluol in US 2014/0039227 A1 beschrieben.

Die Aufreinigung erfolgt im Allgemeinen in einem mehrstufigen Waschverfahren umfassend mindestens drei Waschschritte: einen Waschschritt zur Entfernung der Säuren, einen Waschschritt in Gegenwart einer Base zum Entfernen schwacher Säuren sowie einen neutralen Waschschritt zum Entfernen verbliebener alkalischer Substanzen.

Die resultierenden Waschlösungen können beispielsweise Nitrokresole oder Nitroaromaten enthalten, welche beispielsweise abgetrennt werden können durch Fällung mittels Ansäuern, durch Behandlung mit Aktivkohle, mittels basischer Austauschersäulen, durch Extraktion mit Toluol oder dem zu nitrierenden Aromaten, durch Oxidation mit Salpetersäure und anschließender thermischer Zersetzung, durch Zersetzung mit Wasserstoffperoxid oder Ozon, oder durch Thermolyse der Nitroaromaten.

Nach der Abtrennung von Nitriersäure sowie potentiell vorhandener Nitrokresole und/oder Nitrobenzoesäuren kann die Aufreinigung der Nitroaromaten, wie in US 2014/0039227 A1 für Dinitrotoluol beschrieben, auch in zwei Waschschritten erfolgen. In einem ersten Waschschritt umfassend mindestens einen Extraktionsschritt kann die Rohmischung mit einer ersten Waschsäure enthaltend Salpetersäure, Stickstoffoxide und Schwefelsäure gewaschen werden. Im Allgemeinen hat die bei der Extraktion abgeführte Waschlösung einen Gesamtgehalt an Säure von 20 bis 40 Gew.-%, bezogen auf das Gesamtgewicht dieser Waschlösung, und enthält beispielsweise Salpetersäure, Stickstoffoxide, salpetrige Säure und Schwefelsäure.

Die so verbliebene Mischung, welche die gewünschte Nitroverbindung enthält, wird beispielsweise in einem zweiten Waschschritt mit einer zweiten Waschsäure in mindestens einem weiteren Extraktionsschritt behandelt. Bei diesem Extraktionsschritt hat die abgeführte Waschlösung im Allgemeinen einen pH-Wert von ≤ 4. Üblicherweise wird eine Mischung enthaltend die gewünschte Nitroverbindung erhalten, die grundsätzlich frei von Salpetersäure, Schwefelsäure und Stickstoffoxiden sein kann. Die auf diese Weise erhaltene aufgereinigte Nitroverbindung kann im Allgemeinen zur Hydrierung zu dem entsprechenden Amin verwendet werden.

Die aus dem oben genannten Aufreinigungsverfahren erhaltene oder die zur Hydrierung verwendete Nitroverbindung hat im Allgemeinen einen Restsäuregehalt von ≤ 300 ppm, wie beispielsweise Schwefelsäure, und einen pH-Wert von 2 bis 4. Im Allgemeinen enthält die Nitroverbindung, wie beispielsweise Dinitrotoluol, ≤ 800 ppm Nitrokresole oder Nitrophenole oder Gemische daraus, ≤ 600 pm Nitrobenzoesäure, wie z.B. Mononitrobenzoesäure oder Dinitrobenzoesäure oder Gemische daraus, sowie einen Restgehalt von ≤ 300 ppm, bevorzugt ≤ 200 ppm und besonders bevorzugt ≤ 100 ppm Salpetersäure oder salpetrige Säure oder Gemische daraus, ≤ 50 ppm, bevorzugt ≤ 10 ppm und besonders bevorzugt ≤ 1 ppm Cyanwasserstoffsäure, ≤ 200 ppm, bevorzugt ≤ 50 ppm und besonders bevorzugt ≤ 25 ppm Distickstoffoxid, ≤ 400 ppm, bevorzugt ≤ 200 ppm und besonders bevorzugt ≤ 50 ppm Stickstoffmonoxid, sowie ≤ 3 ppm Sulfat.

In einer bevorzugten Ausführungsform enthält das Reaktionsgemisch neben der zu hydrierenden Nitroverbindung mindestens einen Hochsieder aus der Gruppe bestehend aus Nitrokresolen und Nitrophenolen. In einer besonders bevorzugten Ausführungsform enthält das Reaktionsgemisch neben der zu hydrierenden Nitroverbindung mindestens einen Hochsieder aus der Gruppe bestehend aus Dinitrokresolen, Trinitrokresolen und Nitrophenolen.

In einer anderen bevorzugten Ausführungsform enthält das Reaktionsgemisch neben der zu hydrierende Nitroverbindung keine Hochsieder aus der Gruppe bestehend aus Nitrokresolen und Nitrophenolen. In einer anderen besonders bevorzugten Ausführungsform enthält das Reaktionsgemisch neben der zu hydrierende Nitroverbindung keine Hochsieder aus der Gruppe bestehend aus Dinitrokresolen, Trinitrokresolen und Nitrophenolen

In einer weiteren bevorzugten Ausführungsform enthält das Reaktionsgemisch neben der zu hydrierenden Nitroverbindung mindestens eine Verbindung aus der Gruppe bestehend aus Salpetersäure, Schwefelsäure, Stickstoffoxiden, Distickstoffmonoxid, Cyanwasserstoffsäure, Kohlenstoffmonoxid und Nitrobenzoesäure oder deren Abbauprodukte.

Für das erfindungsgemäße Hydrierverfahren kann die Nitroverbindung in reiner Form, als Mischung mit dem entsprechenden Mono-, Di- oder Polyamin, als Mischung mit dem entsprechenden Mono-, Di- oder Polyamin und Wasser, als Mischung mit dem entsprechenden Mono-, Di- oder Polyamin, Wasser und einem alkoholischen Lösungsmittel oder als Mischung mit dem entsprechenden Di- oder Polyamin, Wasser, einem alkoholischen Lösungsmittel und einem katalysatorreaktivierenden Zusatz eingesetzt werden, wobei jeweils auch Gemische aus zwei oder mehr der oben genannten Nitroverbindungen, der entsprechenden Aminverbindungen, dem alkoholischen Lösungsmittel und dem katalysatorreaktivierenden Zusatz eingesetzt werden können.

Als katalysatorreaktivierende Zusätze werden vorzugsweise aprotische Lösungsmittel, insbesondere, Dimethylformamid (DMF), Dioxan oder Tetrahydrofuran (THF) oder ein Gemisch aus zwei oder mehr davon eingesetzt.

Geeignete alkoholische Lösungsmittel sind im Allgemeinen niedere aliphatische Alkohole mit 1 bis 6 C-Atomen. Bevorzugt werden Methanol, Ethanol oder Propanol einzeln oder in einem Gemisch aus zwei oder mehr davon verwendet. Besonders bevorzugt wird Ethanol verwendet. Sofern ein oben beschriebenes Gemisch eingesetzt wird, liegt das Gewichtsverhältnis von Aminverbindung zu Wasser vorzugsweise im Bereich von 10:1 bis 1:10, bevorzugt im Bereich von 8:1 bis 1:5 und besonders bevorzugt im Bereich von 4:1 bis 1:1 und das Gewichtsverhältnis des Amin/Wasser-Gemischs zu mindestens einem alkoholischen Lösungsmittel vorzugsweise bei 1000:1 bis 1:1, bevorzugt bei 500:1 bis 2,5:1 und besonders bevorzugt bei 50:1 bis 5:1.

Die Menge der eingesetzten alkoholischen Lösungsmittel und der katalysatorreaktivierenden Zusätze ist im Rahmen des erfindungsgemäßen Verfahrens nicht in besonderer Weise beschränkt und kann je nach Bedarf frei gewählt werden.

Das erfindungsgemäße Verfahren zur Hydrierung von Nitroverbindungen zu den entsprechenden Aminen kann zudem in Abwesenheit von Lösungsmitteln durchgeführt werden. Bei dieser Verfahrensweise vereinfacht sich die Aufarbeitung des Reaktionsgemisches nach der Hydrierung, außerdem werden Nebenreaktionen mit dem Lösungsmittel völlig unterbunden.

### Salze

Die aufgereinigte Nitroverbindung kann im Allgemeinen zur Hydrierung zu den entsprechenden Aminen verwendet werden. Die verbliebenen Verunreinigungen sowie die während der Hydrierung entstehenden Nebenprodukte, wie beispielsweise Hochsieder, können jedoch zur Alterung des Katalysators beitragen.

Aus diesem Grund wird die Hydrierung in dem vorliegenden erfindungsgemäßen Verfahren in Gegenwart mindestens eines Salzes der Alkalimetalle, Erdalkalimetalle oder der Seltenerdmetalle durchgeführt.

Bei Seltenerdmetallen handelt es sich im Sinne der Erfindung um Elemente der Gruppe bestehend aus Scandium, Yttrium sowie der Lanthanide. Bei Lanthaniden im Sinne der Erfindung handelt es sich um Elemente der Gruppe bestehend aus Lanthan, Cer, Praseodym, Neodym, Samarium, Europium, Gadolinium, Terbium, Dysprosium, Holmium, Erbium, Thulium, Ytterbium und Lutetium.

Die Hydrierung wird im Allgemeinen in Gegenwart mindestens eines Salzes der Alkali- oder Erdalkalimetalle oder Seltenen Erden oder Gemischen aus diesen, vorzugsweise in Gegenwart mindestens eines Salzes der Alkali- oder Erdalkalimetalle oder Gemischen aus diesen, bevorzugt in Gegenwart mindestens eines Salzes der Alkalimetalle oder Gemischen aus diesen, besonders bevorzugt in Gegenwart mindestens eines Kaliumsalzes oder Natriumsalzes oder Gemischen aus diesen und ganz besonders bevorzugt in Gegenwart mindestens eines Natriumsalzes durchgeführt.

Erfindungsgemäß geeignete Alkalisalze und Erdalkalisalze und Salze der Seltenen Erden enthalten vorzugsweise mindestens ein Element aus der Gruppe bestehend aus Lithium, Natrium, Kalium, Rubidium, Caesium, Magnesium, Calcium, Strontium, Barium, Scandium, Yttrium und den Lanthaniden. Bevorzugt enthält das mindestens eine Salz Kalium, Strontium, Natrium oder Gemische daraus, besonders bevorzugt enthält das mindestens eine Salz Kalium, Natrium oder Gemische daraus.

Vorzugsweise ist das mindestens eine Salz der genannten Alkalimetalle, Erdalkalimetalle und Seltenerdmetalle ein Carbonat, Hydrogencarbonat, Hydroxid, Oxid, Nitrat oder Carboxylat oder ein Gemisch daraus. Geeignete Carboxylate sind zum Beispiel Monocarboxylate und Dicarboxylate, wie Acetat, Formiat und Oxalat. Bevorzugte Salze des erfindungsgemäßen Verfahrens sind Carbonat, Hydrogencarbonat, Nitrat, Acetat, Oxalat, Formiat oder Hydroxid sowie Gemische daraus, besonders bevorzugt sind Carbonat, Hydrogencarbonat, Nitrat oder Hydroxid sowie Gemische daraus. Cyanide und Halogenide sind im Allgemeinen weniger geeignet.

Bevorzugt wird die Hydrierung des erfindungsgemäßen Verfahrens in Gegenwart mindestens eines Salzes ausgewählt aus Carbonat, Hydrogencarbonat, Hydroxid, Oxid, Nitrat und Carboxylat sowie Gemischen daraus, bevorzugt in Gegenwart mindestens eines Salzes ausgewählt aus Carbonat, Hydrogencarbonat, Nitrat oder Hydroxid sowie Gemischen daraus und besonders bevorzugt in Gegenwart mindestens eines Salzes ausgewählt aus Hydrogencarbonat, Nitrat oder Hydroxid sowie Gemischen daraus durchgeführt.

In einer ersten bevorzugten Ausführungsform ist das mindestens eine Salz im Katalysator enthalten. Vorzugsweise liegt das mindestens eine Salz im Katalysator insgesamt in einer Konzentration von 0,05 bis 20 Gew.-%, bevorzugt von 0,05 bis 10 Gew.-% und besonders bevorzugt von 0,1 bis 5 Gew.-%, bezogen auf das Trockengewicht des Katalysators, vor.

In einer zweiten bevorzugten Ausführungsform ist das mindestens eine Salz im flüssigen Reaktionsgemisch enthalten. Vorzugsweise liegt das mindestens eine Salz im flüssigen Reaktionsgemisch insgesamt in einer Konzentration von 0,01 bis 1 mol-%, bevorzugt von 0,02 bis 0,8 mol-% und besonders bevorzugt von 0,03 bis 0,6 mol-%, bezogen auf die zugeführte Menge an zu hydrierender Nitroverbindung, vor.

### Katalysator

Geeignete Trägerkatalysatoren für ein Verfahren zur kontinuierlichen Hydrierung von Nitroverbindungen zu den entsprechenden Aminen in der Flüssigphase sind dem Fachmann allgemein bekannt oder beispielsweise in EP 1 678 118 B1, DE 10 2005 041 532 A1, WO 2008/138784 A1 und US 6,140,539 beschrieben.

Geeignete aktive Komponenten für einen Katalysator zur Hydrierung von Nitroaromaten zu den entsprechenden Aminen sind dem Fachmann allgemein bekannt oder beispielsweise in EP 1 678 118 B1, DE 10 2005 041 532 A1, WO 2008/138784 A1, EP 1161297 A1, EP 1165231 A1 und US 6,140,539 beschrieben.

Im Allgemeinen enthält der Trägerkatalysator als aktive Komponente mindestens ein Element aus den Gruppen 7 bis 12 des Periodensystems der Elemente. Als aktive Komponente des Trägerkatalysators geeignete Elemente aus den Gruppen 7 bis 12 des Periodensystems der Elemente sind beispielsweise Eisen, Kobalt, Nickel, Ruthenium, Rhodium, Palladium, Silber, Osmium, Iridium, Platin, Gold, Kupfer, Rhenium, Zink und/oder Mangan.

Bevorzugt enthält die aktive Komponente des Trägerkatalysators mindestens ein Element aus den Gruppen 7 bis 12 des Periodensystems der Elemente, besonders bevorzugt mindestens ein Element aus der Gruppe bestehend aus Nickel, Platin, Palladium, Eisen und Kobalt, ganz besonders bevorzugt mindestens ein Element aus der Gruppe bestehend aus Nickel, Platin Palladium und Kobalt und insbesondere bevorzugt mindestens Nickel.

Im Allgemeinen enthält der Katalysator 0 bis 10 Gew.-%, bevorzugt 1 bis 5 Gew.-% Edelmetall, bezogen auf das Gesamtgewicht des Katalysators.

Optional kann die aktive Komponente des Katalysators neben dem mindestens einen Element aus den Gruppen 7 bis 12 des Periodensystems der Elemente zusätzlich Chrom enthalten.

In einer bevorzugten Ausführungsform enthält der Trägerkatalysator mindestens ein Salz der Alkalimetalle, Erdalkalimetalle oder der Seltenerdmetalle. Vorzugsweise enthält der Katalysator das mindestens eine Salz insgesamt in einer Konzentration von 0,05 bis 20 Gew.-%, bevorzugt von 0,05 bis 10 Gew.-% und besonders bevorzugt von 0,1 bis 5 Gew.-%, bezogen auf das Trockengewicht des Katalysators.

Um Nebenreaktionen zu unterdrücken, ist es bevorzugt, das Verfahren so zu führen, dass der Katalysator an seiner Belastungsgrenze gefahren wird. Dies kann beispielsweise durch die Menge der zudosierten Nitroverbindung, die Menge des Katalysators im Reaktionsgemisch, die Temperatur oder den Druck gesteuert werden. Unter der Belastungsgrenze des Katalysators im Sinne der Erfindung wird die Menge der hydrierbaren Stickstoffatome und Sauerstoffatome enthaltenden Gruppen verstanden, die vom Katalysator bei gegebenen Druck- und Temperaturbedingungen hydriert werden können. Bei den Stickstoffatome und Sauerstoffatome enthaltenden Gruppen kann es sich neben Nitrogruppen auch um Nitrosogruppen und Nitrosamingruppen handeln.

In einer ersten Ausführungsform kann, wie in US 6,140,539 beschrieben, Nickel als aktive Komponente auf einem Träger verwendet werden, wobei der Katalysator stabilisiert ist, und die Nickelkristallite eine bimodale Nickelkristallitgrößenverteilung, einen Nickel-Gehalt von 60 bis 80 Gew.-%, bezogen auf die Gesamtmasse des Katalysators, sowie einen Reduktionsgrad von mindestens 70% aufweisen.

Die Bestimmung des Reduktionsgrades erfolgt im Allgemeinen bei einer einstündigen Nachreduktion des stabilisierten Katalysators bei 100°C.

Im Allgemeinen liegen die beiden Maxima der bimodalen Nickelkristallitgrößenverteilung bei 30 bis 80 Angström und 81 bis 150 Angström. Bevorzugt beträgt der Anteil des Nickels im Bereich des Maximums von 30 bis 80 Angtröm von ≥ 40 bis <100 Gew.-% bezogen auf die Gesamtmasse des Katalysators.

Als Trägermaterial dieser ersten Ausführungsform sind beispielsweise Oxide und Oxidgemische von Zirkonium, Hafnium oder Silizium geeignet. Vorzugsweise enthält der Träger ZrO₂, ZrO₂HfO₂, SiO₂·ZrO₂ oder SiO₂·ZrO₂HfO₂ oder Gemische enthaltend mindestens zwei dieser Substanzen. Besonders bevorzugt besteht der Träger aus diesen Substanzen.

Bevorzugt beträgt der SiO₂-Gehalt 0 bis 20 Gew.-%, bezogen auf die Gesamtmasse des Katalysators.

Vorzugweise beträgt der ZrO₂- Gehalt 0 bis 40 Gew.-%, bezogen auf die Gesamtmasse des Katalysators.

Bevorzugt beträgt der HfO₂-Gehalt 0 bis 4 Gew.-%, bezogen auf die Gesamtmasse des Katalysators.

Verfahren zur Herstellung eines solchen Katalysators sind dem Fachmann allgemein bekannt oder beispielsweise in US 6,140,539 beschrieben.

Vorzugsweise enthält die aktive Komponente des Trägerkatalysators Nickel in Form von Nickelkristalliten mit einer bimodalen Nickelkristallitgrößenverteilung und weist einen Nickel-Gehalt von 60 bis 80 Gew.-% bezogen auf die Gesamtmasse des Katalysators sowie einen Reduktionsgrad von mindestens 70% auf.

In einer zweiten Ausführungsform wird in dem erfindungsgemäßen Verfahren zur kontinuierlichen Hydrierung von Nitroverbindungen zu den entsprechenden Aminen ein Trägerkatalysator verwendet, dessen aktive Komponente ein Gemisch aus Nickel und Platin sowie gegebenenfalls mindestens eine zusätzliches Metall enthält, wie beispielsweise in EP 1678118 B1 oder DE 10 2005 041 532 A1 beschrieben.

Im Allgemeinen werden aktive Komponenten dieses Katalysators in Form von Gemischen auf das Trägermaterial aufgebracht. Geeignete Gemische enthalten Nickel und Platin mit einem Atomverhältnis von Nickel zu Platin von vorzugsweise zwischen 30:70 und 70:30, bevorzugt zwischen 40:60 und 60:40 und besonders bevorzugt zwischen 45:55 und 55:45. Gemische aus Nickel und Platin mit einem anderen Atomverhältnis sind ebenfalls brauchbar, führen jedoch häufig zu geringen Produktausbeuten.

Bevorzugt wird dem Gemisch enthaltend Nickel und Platin noch mindestens ein zusätzliches Metall hinzugefügt. Als zusätzliches Metall geeignete Metalle sind dem Fachmann allgemein bekannt oder beispielsweise in DE 10 2005 041 532 A1 beschrieben. Bevorzugt ist das zusätzliche Metall mindestens ein Metall aus der Gruppe bestehend aus Kupfer, Kobalt, Eisen, Zink, Mangan und Chrom, besonders bevorzugt mindestens ein Metall aus der Gruppe bestehend aus Kupfer, Kobalt, Eisen und Zink.

Die Metallpartikel sind im Allgemeinen polykristallin. Ihre Charakterisierung ist dem Fachmann allgemein bekannt oder beispielsweise in DE 10 2005 041 532 A1 beschrieben.

Die Beschaffenheit sowie Verfahren zur Charakterisierung des Trägerkatalysators, bei dessen aktivem Material es sich um ein Gemisch aus Nickel und Platin sowie gegebenenfalls mindestens einem zusätzlichen Metall handeln kann, sind dem Fachmann allgemein bekannt oder in EP 1678118 B1 oder DE 10 2005 041 532 A1 beschrieben.

Im Allgemeinen enthält der im erfindungsgemäßen Verfahren verwendete Hydrierkatalysator basierend auf Nickel und Platin und mindestens einem zusätzlichen Metall
1 bis 5 Gew.-% Platin,
0,3 bis 1,5 Gew.-% Nickel,
0,05 bis 1,5 Gew.-% des mindestens einen zusätzlichen Metalls, sowie
94,65 bis 97,45 Gew.-% Trägermaterial,
jeweils bezogen auf das Gesamtgewicht des Katalysators, wobei die Summe 100 Gew.-% ergibt.

Besonders bevorzugt besteht der im erfindungsgemäßen Verfahren verwendete Hydrierkatalysator basierend auf Nickel und Platin und mindestens einem zusätzlichen Metall aus
1 bis 5 Gew.-% Platin,
0,3 bis 1,5 Gew.-% Nickel,
0,05 bis 1,5 Gew.-% des mindestens einen zusätzlichen Metalls, sowie
94,65 bis 97,45 Gew.-% Trägermaterial,
jeweils bezogen auf das Gesamtgewicht des Katalysators, wobei die Summe 100 Gew.-% ergibt.

Im Allgemeinen beträgt der Gehalt an Nichtedelmetallen 0 bis 1,6 Gew.-%, bevorzugt 0,1 bis 0,9 Gew.-%, bezogen auf das Gesamtgewicht des Katalysators.

Die als Träger für die Katalysatoren dieser zweiten Ausführungsform geeigneten Materialen sind dem Fachmann allgemein bekannt oder beispielsweise in EP 1678118 B1 oder DE 10 2005 041 532 A1 beschrieben. Im Allgemeinen werden Aktivkohle, Ruß, Graphit oder Metalloxide, bevorzugt hydrothermal stabile Metalloxide, wie z.B. ZrO₂ und/oder TiO₂, oder Oxide des Aluminiums, wie Al₂O₃, oder Siliciums oder anderer Materialien eingesetzt.

Vorzugsweise wird Graphit als Träger verwendet, wobei in diesem Fall HSAG (High Surface Area Graphite) mit einer Oberfläche von 50 bis 300 m²/g besonders bevorzugt sind.

Besonders bevorzugt ist die Verwendung von Aktivkohle als Träger. Eine ganz besonders bevorzugte Ausführungsform ist die Verwendung von physisch oder chemisch aktivierter Aktivkohle oder Ruße, wie Acetylenruß, als Träger.

Verfahren zur Herstellung des Trägerkatalysators, bei dessen aktivem Material es sich um ein Gemisch aus Nickel und Platin sowie gegebenenfalls mindestens einem zusätzlichen Metall handelt, sind dem Fachmann allgemein bekannt oder in EP 1678118 B1 oder DE 10 2005 041 532 A1 beschrieben.

Der im erfindungsgemäßen Verfahren verwendete Katalysator basierend auf einem Gemisch aus Nickel und Platin und gegebenenfalls mindestens einem zusätzlichen Metall wird vorzugsweise in einer Menge von 0,01 bis 10 Gew.-%, bevorzugt 0,1 bis 5 Gew.-% und besonders bevorzugt 0,2 bis 2 Gew.-%, bezogen auf das Gesamtgewicht des Reaktionsgemisches, eingesetzt.

Bevorzugt enthält die aktive Komponente des Trägerkatalysators ein Gemisch aus Nickel und Platin sowie gegebenenfalls mindestens einem zusätzlichen Metall.

Bevorzugt ist das zusätzliche Metall mindestens ein Metall aus der Gruppe bestehend aus Kupfer, Kobalt, Eisen, Zink, Mangan und Chrom, besonders bevorzugt mindestens ein Metall aus der Gruppe bestehend aus Kupfer, Kobalt, Eisen und Zink.

Gemäß einer dritten Ausführungsform des erfindungsgemäßen Verfahrens können Katalysatoren eingesetzt werden, die als aktive Komponente ein Gemisch von Nickel, Palladium und einem zusätzlichen Element, ausgewählt aus der Gruppe, bestehend aus Kobalt, Eisen, Vanadium, Mangan, Chrom, Platin, Iridium, Gold, Bismut, Molybdän, Selen, Tellur, Zinn und Antimon auf einem Träger enthalten. Dieses Verfahren kann insbesondere zur Herstellung von Toluylendiamin durch Hydrierung von Dinitrotoluol angewandt werden. Die Durchführung einer Hydrierung von Nitroverbindungen zu den entsprechenden Aminen mithilfe dieses Katalysators ist dem Fachmann allgemein bekannt oder beispielsweise in der WO 2008/138784 A1 beschrieben.

Das zusätzliche Element ist vorzugsweise ausgewählt aus der Gruppe, bestehend aus Kobalt, Eisen, Vanadium, Bismut und Zinn.

Als Träger für den Katalysator dieser dritten Ausführungsform können im Allgemeinen die hierfür üblichen und bekannten Materialien eingesetzt werden. Vorzugsweise werden Aktivkohle, Ruß, Graphit oder Metalloxide, bevorzugt hydrothermal stabile Metalloxide wie z.B. ZrO₂, TiO₂, Al₂O₃ eingesetzt. Bei Graphit sind die HSAG (High Surface Area Graphite) mit einer Oberfläche von 50 bis 300 m²/g bevorzugt. Besonders bevorzugt sind Aktivkohlen, insbesondere physikalisch oder chemisch aktivierte Aktivkohlen, oder Ruße, wie Acetylenruß.

In einer weiteren Ausführungsform des erfindungsgemäßen Verfahrens ist die aktive Komponente des Katalysators kein Raney-Nickel.

Im Allgemeinen wird der im erfindungsgemäßen Verfahren verwendete Katalysator in einer Menge von 0,01 bis 10 Gew.-%, bevorzugt 0,1 bis 5 Gew.-% und besonders bevorzugt 0,2 bis 2 Gew.-%, bezogen auf das Gesamtgewicht des Reaktionsgemisches, eingesetzt.

Der Katalysator wird üblicherweise in reduziertem und passiviertem Zustand in den Reaktor eingebracht. Unter dem reduzierten und passiviertem Zustand im Sinne der Erfindung wird verstanden, dass der Katalysator nach der Herstellung aktiviert ist, danach jedoch aus Sicherheitsgründen die aktiven Zentren beispielsweise durch Überleiten von Sauerstoff oder Kohlendioxid passiviert werden. Geeignet sind zudem der Ausbau und die Stabilisierung des Katalysators unter einer inerten Atmosphäre oder in einem nicht leicht-entzündlichen Lösungsmittel, beispielsweise in Wasser oder einem Gemisch aus Toluendiamin und Wasser, oder höheren Alkoholen, wie z.B. Butanol oder Ethylenglykol.

Ein weiterer Gegenstand der Erfindung ist ein Trägerkatalysator zur kontinuierlichen Hydrierung von einer Nitroverbindung zu dem entsprechenden Amin in einem die Nitroverbindung enthaltenden flüssigen Reaktionsgemisch, der als aktive Komponente mindestens ein Element aus den Gruppen 7 bis 12 des Periodensystems der Elemente enthält, und zusätzlich mindestens ein Salz der Alkalimetalle, Erdalkalimetalle oder der Seltenerdmetalle enthält.

### Durchführung

Die für das erfindungsgemäße Verfahren geeigneten Reaktoren und Reaktorfahrweisen sind dem Fachmann allgemein bekannt oder beispielsweise in DE10 2005 041 532 A1, DE 10 2008 063 308 B4, WO 2000/035852 A1 oder WO 2014/108351 A1 beschrieben.

Die für das erfindungsgemäße Verfahren anzuwendenden Verfahrensparameter, wie Druck und Temperatur, sind dem Fachmann ebenfalls allgemein bekannt oder beispielsweise in DE 10 2005 041 532 A1, DE 10 2008 063 308 B4, WO 2000/035852 A1 oder WO 2014/108351 A1 beschrieben.

Geeignete Reaktoren sind beispielsweise Rührkessel oder Rohrbündelreaktoren oder Schlaufenreaktoren, wie Strahlschlaufenreaktoren, sogenannte Loop-Venturi-Reaktoren, oder Schlaufenreaktoren mit innerer Strömungsumkehr, wie in WO 2000/035852 A1, DE10 2005 041 532 A1, DE 10 2008 063 308 B4 oder WO 2014/108351 A1 beschrieben. Bevorzugt wird ein Schlaufenreaktor für das erfindungsgemäße Verfahren verwendet.

Im Allgemeinen werden die Nitroverbindungen mit einer Rate zugegeben, die einerseits der Katalysatoraktivität angepasst ist und andererseits eine ausreichende Vermischung mit der Strömung im Schlaufenreaktor, dem sogenannten Zirkulationsstrom, ergibt. In der Regel wird die Katalysatoraktivität durch Zugabe ausreichender Katalysatormengen so eingestellt, dass sich der Zugabestrom der Nitroverbindungen nach dem Zirkulationsstrom richtet, so dass lokale Überkonzentrationen von Nitroverbindungen (zum Beispiel größer 10.000 ppm) vermieden werden. Im Fall eines Reaktors mit Strömungsumkehr kann beispielsweise durch den Aufprall des eingespeisten Reaktionsgemischs auf den Reaktorboden oder durch Einbauten eine interne Zirkulationsströmung entstehen, die größer als der Zirkulationsstrom ist. Wird die Nitroverbindung in diesem Fall in die interne Zirkulationsströmung gegeben, richtet sich ihr Zugabestrom nach der Größe der internen Zirkulationsströmung, um bei gegebenem Umsatz in der internen Zirkulationsschlaufe lokale Überkonzentrationen zu vermeiden.

Kurz nach der ersten Zugabestelle folgt im Allgemeinen eine zweite Zugabestelle, durch die zusätzliche Komponenten, wie beispielsweise Salze, in das Verfahren eingespeist werden können.

Die Salze der Alkalimetalle, Erdalkalimetalle oder der Seltenerdmetalle können im Allgemeinen an beliebiger Stelle in den Reaktor eingespeist werden. Vorzugweise werden sie nicht in die dem Reaktor zugeführten Nitroverbindungen gegeben.

Die in dem erfindungsgemäßen Verfahren angewandte gewichtsbezogene Raumgeschwindigkeit beträgt vorzugsweise 5 bis 100 kg (Nitroverbindung)/kg (Katalysator)/h, bevorzugt 10 bis 50 kg (Nitroverbindung)/kg (Katalysator)/h und besonders bevorzugt 15 bis 35 kg (Nitroverbindung)/kg (Katalysator)/h.

Geeignete Hydriergasgemische zur kontinuierlichen Hydrierung von Nitroaromaten zu den entsprechenden Aminen sind dem Fachmann allgemein bekannt oder beispielsweise in EP 1678 118 B1 beschrieben.

Im Allgemeinen können als Hydriergasgemische Gase verwendet werden, die freien Wasserstoff enthalten und keine schädlichen Mengen an Katalysatorgifte, wie beispielsweise Kohlenmonoxid, enthalten. Geeignete Hydriergasgemische sind Reformerabgase, oder Gemische aus Wasserstoff mit Stickstoff und/oder Kohlendioxid. Bevorzugt wird Wasserstoff mit geringem Inertgasanteil als Hydriergasgemisch verwendet.

Die Zufuhr der Hydriergasgemische erfolgt im Allgemeinen abhängig von dem Wasserstoffverbrauch durch die Reaktion, indem der in der Reaktorapparatur herrschende Druck vorzugsweise kontant gehalten wird. Durch kontinuierliche Entnahme eines Teils der Gasphase kann eine Aufpegelung von Inertanteilen im Hydriergasgemisch oder von inerten gasförmigen Reaktionsprodukten vermieden werden.

In einer ersten Ausführungsform kann das erfindungsgemäßen Verfahren, wie in WO 2014/108351 A1 oder DE 10 2008 063 308 B4 beschrieben, bei Temperaturen von 80 bis 200°C, bevorzugt von 110 bis 190°C, besonders bevorzugt von 150 bis 190°C durchgeführt werden. Im Allgemeinen wird das Verfahren dieser ersten Ausführungsform bei Drücken von 10 bis 50 bar, bevorzugt von 15 bis 35 bar durchgeführt. Die dabei entstehende Reaktionswärme kann mit Hilfe von Wärmeüberträgern zur Gewinnung von Dampf mit einem Überdruck von mindestens 4 bar genutzt werden. Die für die Durchführung dieses Verfahrens benötigten Parameter sind dem Fachmann allgemein bekannt oder beispielsweise in WO 2014/108351 A1 oder DE 10 2008 063 308 B4 beschrieben.

Vorzugsweise wird das erfindungsgemäße Verfahren zur Hydrierung von Nitroverbindungen zu Aminen, wie beispielsweise die Hydrierung von Dinitrotoluol zu Toluylendiaminderivaten, in einem vertikal ausgedehnten, wie in WO 2014/108351 A1 beschriebenen, Reaktor durchgeführt, welcher mindestens eine Mischkammer enthält, wobei die Mischkammer oder die Mischkammern an ihrem unteren Ende jeweils mit einem Diffusor verbunden sind.

Für dieses Verfahren geeignete Wärmeüberträger und Kühlmedien sind dem Fachmann ebenfalls allgemeinen bekannt oder beispielsweise in WO 2014/108351 A1 oder DE 10 2008 063 308 B4 beschrieben.

In einer zweiten Ausführungsform kann das erfindungsgemäßen Verfahren, wie in WO 00/35852 A1 oder DE 10 2005 041 532 A1 beschrieben, bei Temperaturen von vorzugsweise 80 bis 250°C, bevorzugt von 100 bis 200°C und besonders bevorzugt von 120 bis 150°C durchgeführt werden. Im Allgemeinen wird das Verfahren in dieser zweiten Ausführungsform bei Drücken von vorzugsweise 5 bis 100 bar, bevorzugt von 10 bis 40 bar und besonders bevorzugt von 20 bis 25 bar durchgeführt.

Geeignete Reaktoren sind dem Fachmann allgemein bekannt oder beispielsweise in WO 00/35852 A1 oder DE 10 2005 041 532 A1 beschrieben. Im Allgemeinen können Rührkessel oder Schlaufenreaktoren, wie Strahlschlaufenreaktoren, sogenannte Loop-Venturi-Reaktoren, oder Schlaufenreaktoren mit innerer Strömungsumkehr eingesetzt werden.

Im Allgemeinen können die bei der Hydrierung gebildeten Amine dem Verfahren kontinuierlich oder diskontinuierlich entnommen werden. Bevorzugt werden die bei der Hydrierung gebildeten Amine dem Verfahren kontinuierlich entnommen.

Die Entnahme der bei der Hydrierung gebildeten Amine kann an beliebiger Stelle erfolgen. Bevorzugt erfolgt die Entnahme aus der externen Zirkulationsströmung vor der Einspeisung der Nitroverbindung. Da die Hydrierung der Nitroverbindung unter den genannten Bedingungen in der internen Zirkulationsströmung im Allgemeinen praktisch quantitativ abläuft, enthält die externe Zirkulationsströmung vor der Einspeisung der Nitroverbindung im Wesentlichen das entsprechende reine Amin, Wasser, gegebenenfalls Lösungsmittel und Katalysator. Das Amin wird im Allgemeinen aus dem abgezogenen Strom abgetrennt und der Reinigung zugeführt. Der Katalysator und gegebenenfalls Wasser können der externen Zirkulationsströmung wieder zugeführt werden.

Vorzugsweise wird der Katalysator in dem Reaktionsmedium suspendiert. Die Abtrennung des Reaktionsprodukts vom Katalysator erfolgt im Allgemeinen mittels Membranfiltration. Die dazu genutzte Membran kann bevorzugt in der äußeren Umlaufströmung oder beispielsweise in den kontinuierlich gerührten Rührkessel eingebaut werden. Alternativ kann der Katalysator auch durch Sedimentation in einem Settler zurückgehalten werden, der in die äußere Umlaufströmung oder beispielsweise in den kontinuierlich gerührten Rührkessel eingebaut ist.

Die Membranfiltration wird vorzugsweise bei einem Druck auf der Suspensionsseite von 5 bis 50 bar, bevorzugt von 20 bis 35 bar, einer Druckdifferenz zwischen der Suspensionsseite und der Permeatseite von 0,3 bar bis 5 bar und einer Strömungsgeschwindigkeit auf der Suspensionsseite von 1 bis 6 m/s durchgeführt. Unter Suspensionsseite im Sinne der Erfindung wird die Seite des Membranfilters verstanden, auf der sich die Katalysator enthaltende Mischung befindet. Unter Permeatseite im Sinne der Erfindung wird die Seite des Membranfilters verstanden, auf der sich die katalysatorfreie Mischung befindet.

Die Membranfiltration kann kontinuierlich oder diskontinuierlich durchgeführt werden.

Bei der kontinuierlichen Durchführung wird im Allgemeinen ständig zumindest ein Teilstrom des Reaktionsgemisches durch einen Membranfilter gefahren. In einer bevorzugten Ausführungsform des erfindungsgemäßen Verfahrens ist es bevorzugt, den Membranfilter in dem externen Kreislauf eines Umlaufreaktors anzuordnen.

Bei der diskontinuierlichen Durchführung der Filtration wird das ausgeschleuste Reaktionsgemisch im Allgemeinen durch eine zuschaltbare Reinigungsstufe, bestehend aus mindestens einem Membranfilter und einer eigenen Zirkulationspumpe, geleitet. Bei einer anderen Ausgestaltung der diskontinuierlichen Filtration wird im Anschluss an die Reaktion das Reaktionsgemisch über einen Membranfilter gefahren.

Die für das Verfahren eingesetzten Filtermembranen können beispielsweise aus Keramik (z.B. α-Al₂O₃) oder Edelstahl (z.B. 1.4404) bestehen und haben, abhängig von der Partikelgröße des eingesetzten Katalysators, bevorzugt zahlengewichtete mittlere Porendurchmesser im Bereich von 10 nm bis 20 Mikrometer, besonders bevorzugt im Bereich von 20 nm bis 10 Mikrometer und ganz besonders bevorzugt von 50 nm bis 1 Mikrometer.

Geeignete Ausführungsformen einer Membranfiltration, insbesondere Querstromfiltration, sind dem Fachmann bekannt und werden beispielsweise in der WO2010/125025 oder WO 2003/066571 beschrieben.

Nach der Abtrennung des Katalysators werden die gebildeten Amine im Allgemeinen weiter aufgereinigt. Verfahren zur Aufreinigung der erfindungsgemäß hergestellten Amine sind dem Fachmann allgemein bekannt oder beispielsweise in WO 2000/035852 A1 beschrieben. Geeignete Verfahren zur Aufreinigung der gebildeten Amine sind z.B. Destillation oder Extraktion.

### Beschreibung der Abbildungen

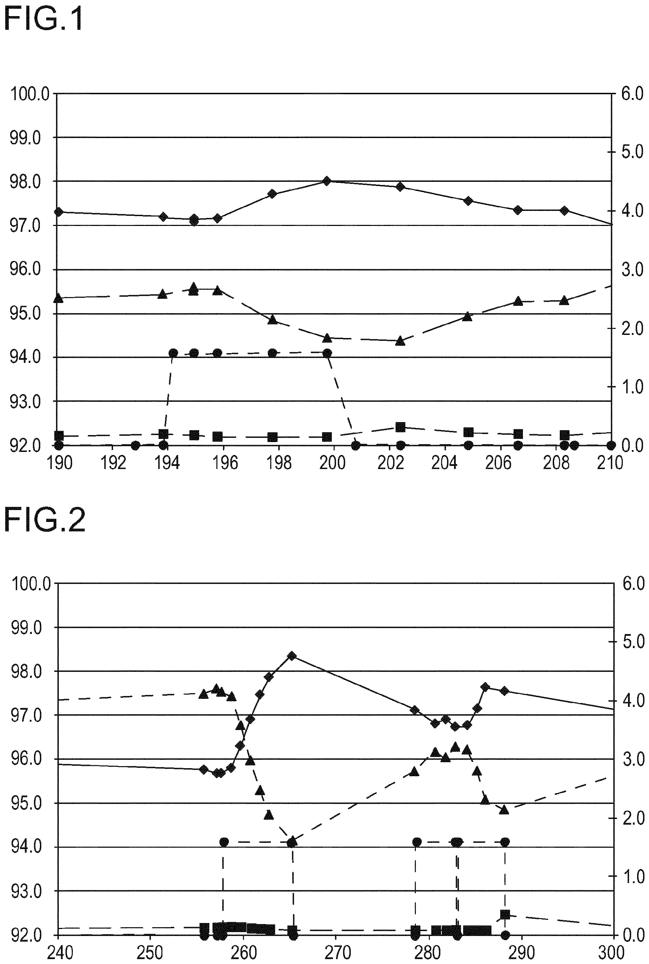

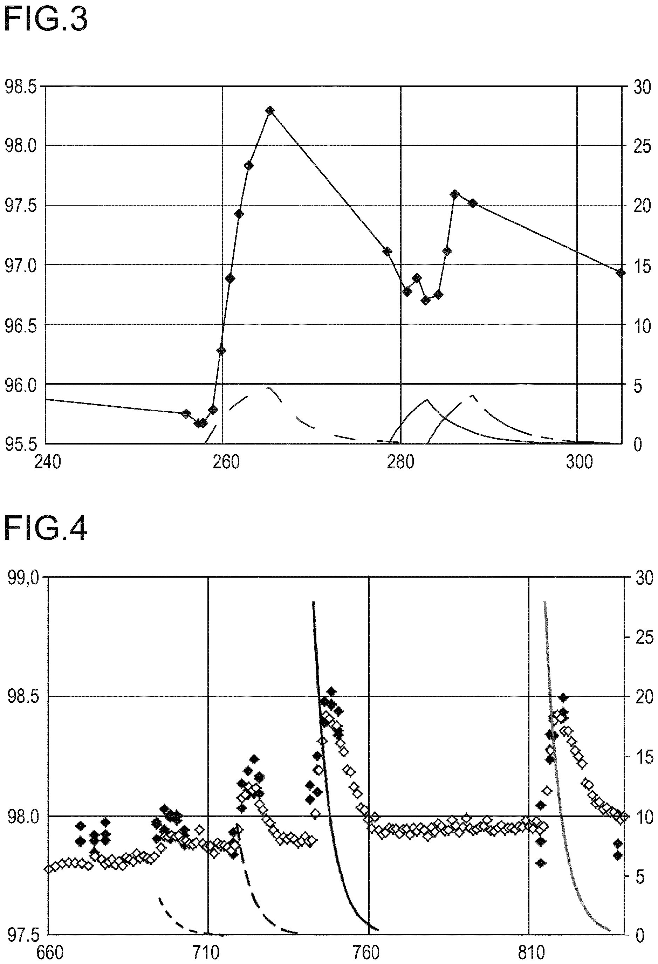

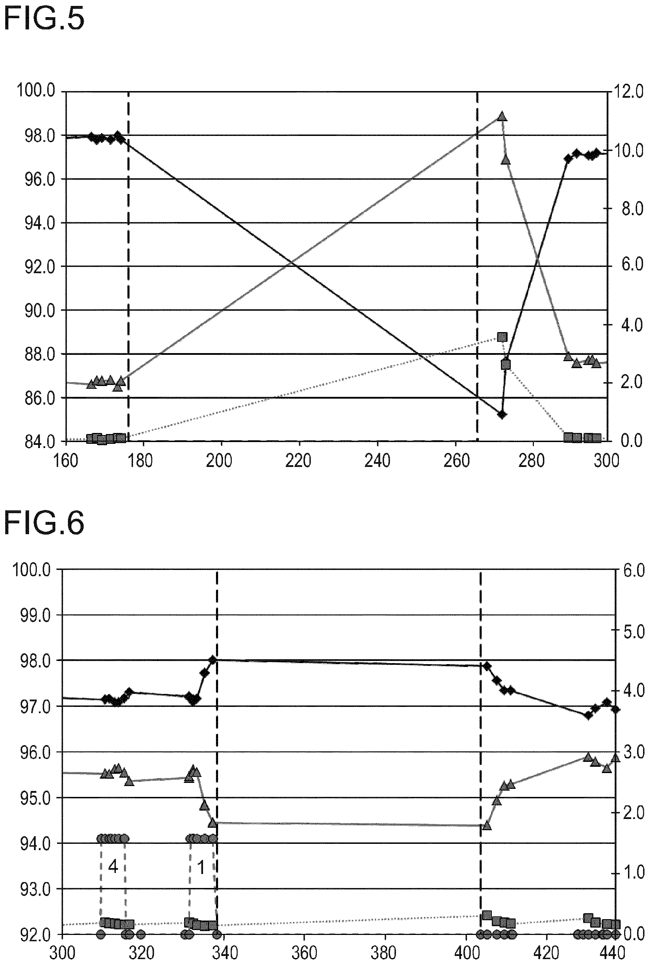

In FIG. 1 bis FIG. 4 und FIG.7 beschreibt die x-Achse des Diagramms die Laufzeit der Reaktion in Stunden, während DNT zudosiert wird. In FIG. 5 bis FIG. 6 beschreibt die x-Achse des Diagramms die Laufzeit des Versuchs in Stunden seit der ersten DNT-Zudosierung (inklusive Unterbrechungen der Reaktion). Die linke y-Achse zeigt die TDA-Ausbeute in Flächen-% bezogen auf die Gesamtfläche aller Peaks bei der Gaschromatographie (GC) an. Bei FIG. 1, 2, 5, 6 und 7 gibt die rechte y-Achse die Ausbeute an niedrig bzw. hoch siedenden Komponenten in Flächen-% und den Co-Dosierungsfluss in Zentilitern/Stunde an. In FIG. 3 und 4 beschreibt die rechte y-Achse die berechnete Kationenkonzentration (ideale Rückvermischung und Löslichkeit bzw. kontinuierlichen proportionalen Austrag) in mmol/kg. Die Kationenkonzentrationen sind für vollständige Rückvermischung und kontinuierlichen Austrag berechnet.
FIG. 1 gibt die TDA-, Leicht- und Hochsiederausbeuten bei Zudosierung einer wässrigen Lösung mit 4000 ppm Kaliumbicarbonat (resultierend in eine Konzentration von 552 ppm Kaliumbicarbonat) wieder.
   Bei den Kurven repräsentieren die Datenpunkte ◆: Ausbeute an TDA, □: Ausbeute an niedrig siedenden Komponenten, Δ: Ausbeute an hoch siedenden Komponenten und o: Co-Dosierungsfluss an KHCO₃.
   Die senkrechte gestrichelte Linie markiert eine Unterbrechung der DNT-Zufuhr über einen Zeitraum von fast 70h, bei der der Zirkulationsstrom und alle anderen Bedingungen aufrechterhalten bleiben.
FIG. 2 gibt die TDA-, Leicht- und Hochsiederausbeuten bei Zudosierung wässriger Lösungen von Kaliumbicarbonat, Ammoniumbicarbonat und Kaliumhydroxid wieder.
   Bei den Kurven geben die Datenpunkte ◆: Ausbeute an TDA, □: Ausbeute an niedrig siedenden Komponenten, Δ: Ausbeute an hoch siedenden Komponenten und ○: Co-Dosierungsstrom an. Der Co-Dosierungsstrom besteht nacheinander aus 4000 ppm Kaliumbicarbonat (1), 3150 ppm Ammoniumbicarbonat (2) und 2250 ppm Kaliumhydroxid (3) (jeweils wässrige Lösungen).
FIG. 3 gibt die TDA-Ausbeute bei Zudosierung wässriger Lösungen verschiedener Salze wieder.
   Die ◆ Datenpunkte geben die Ausbeute an TDA an. Die gestrichelte Kurve (links) beschreibt die berechnete Kaliumkonzentration ausgehend von Kaliumbicarbonat (Endkonzentration 552 Gewichts-ppm (wppm)), die durchgehende Kurve (mitte) die berechnete Ammoniumkonzentration ausgehend von Ammoniumbicarbonat (Endkonzentration 434 wppm) und die gepunktetgestrichelte Linie (rechts) die berechnete Kaliumkonzentration ausgehend von Kaliumhydroxid (Endkonzentration 310 wppm) im Reaktorstrom.
FIG. 4 gibt die TDA-Ausbeute bei pulsweiser Zudosierung wässriger Lösungen verschiedener Salze wieder.
   Mit einem ◆ markierte Datensätze beschreiben die Ausbeute an TDA in Flächen-% bezogen auf die Gesamtfläche aller Peaks der GC. Durch ein ◊ markierte Datenwerte beschreiben die Ausbeute an TDA in Flächen-% bezogen auf die Gesamtfläche aller Peaks, wie sie in einem integrierten online-GC bestimmt werden.
   Die Kurven der Kationenkonzentration bzw. Kaliumkonzentration oder Natriumkonzentration (von links nach rechts) resultieren aus einer Startkonzentration von 121 wppm Kaliumbicarbonat (gestrichelt, kurze Striche), einer Startkonzentration von 363 wppm Kaliumbicarbonat (gestrichelt, lange Striche), einer Startkonzentration von 1089 wppm Kaliumbicarbonat (durchgehend, schwarz) und einer Startkonzentration von 642 wppm Natriumbicarbonat (durchgehend, grau) im Reaktorstrom.
   Die Kationenkonzentrationen sind für vollständige Rückvermischung und kontinuierlichen Austrag berechnet.
FIG. 5 gibt die TDA-, Leicht- und Hochsiederausbeuten bei Unterbrechung der DNT-Dosierung in Abwesenheit von Kaliumbicarbonat wieder.
   Datenpunkte gekennzeichnet durch: ◆ repräsentieren die Ausbeute an TDA, □ zeigen die Ausbeute an niedrig siedenden Komponenten an und Δ stellen die Ausbeute an hoch siedenden Komponenten dar.
   Die senkrecht gestrichelten Linien markieren eine Unterbrechung der DNT-Zufuhr über einen Zeitraum von 90h, bei der der Zirkulationsstrom und alle anderen Bedingungen aufrechterhalten bleiben.
FIG. 6 gibt die TDA-, Leicht- und Hochsiederausbeuten bei Unterbrechung der DNT-Dosierung in Gegenwart von Kaliumbicarbonat (Zudosierung vor Unterbrechung) wieder.
   Bei den Kurven geben die Datenpunkte ◆: Ausbeute an TDA, □: Ausbeute an niedrig siedenden Komponenten, Δ: Ausbeute an hoch siedenden Komponenten und ○: Co-Dosierungsstrom an. Der Co-Dosierungsstrom besteht nacheinander aus H₂O (4) und 4000 ppm wässrige Kaliumbicarbonatlösung (1).
   Die senkrecht gestrichelten Linien markieren eine Unterbrechung der DNT-Zufuhr über einen Zeitraum von fast 70h, bei der der Zirkulationsstrom und alle anderen Bedingungen aufrechterhalten bleiben.

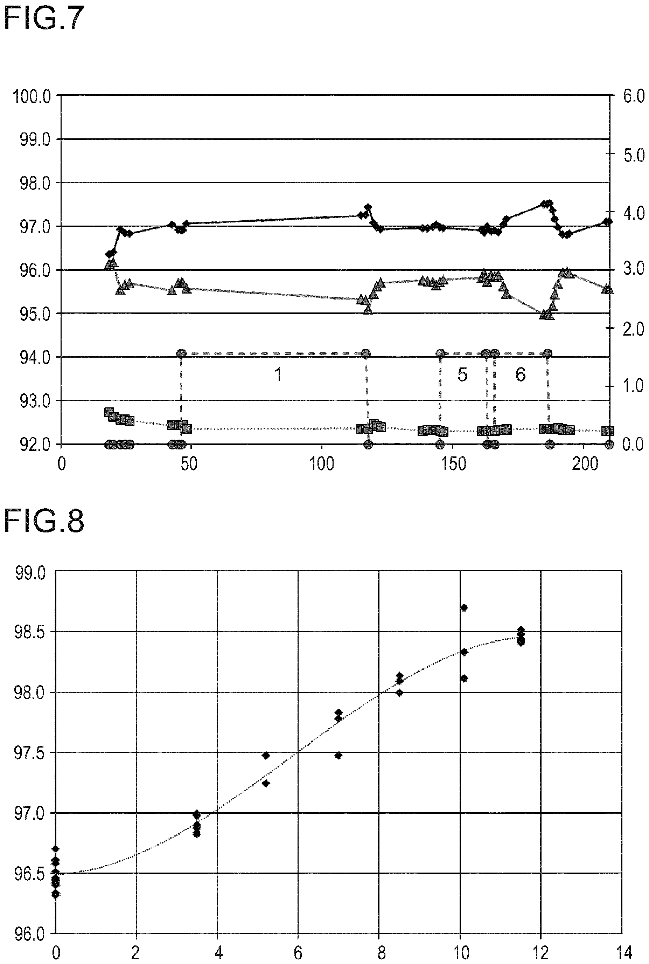

FIG. 7 gibt die TDA-Ausbeuten, Leichtsiederausbeuten und Hochsiederausbeuten bei Zudosierung wässriger Lösungen von Kaliumbicarbonat und Strontiumnitrat wieder.
   Bei den Kurven geben die Datenpunkte ◆: Ausbeute an TDA, □: Ausbeute an niedrig siedenden Komponenten, Δ: Ausbeute an hoch siedenden Komponenten und ○: Co-Dosierungsstrom an. Der Co-Dosierungsstrom besteht nacheinander aus 4000 ppm Kaliumbicarbonat (1) (Endkonzentration 5,5mmol(K)/kg), 1600 ppm Strontiumnitrat (5) (Endkonzentration 1,0 mmol(Sr)/kg) und 10 000 ppm Strontiumnitrat (6) (Endkonzentration 6,5mmol(Sr)/kg) (jeweils wässrige Lösungen).
FIG. 8 gibt die TDA-Ausbeute bei Zudosierung steigender Kalium-Konzentrationen
   Die x-Achse des Diagramms beschreibt die Kalium-Konzentration im Reaktor in mmol/kg. Die y-Achse zeigt die TDA-Ausbeute in Flächen-% bezogen auf die Gesamtfläche aller Peaks bei der GC.
   Die Datenpunkte ◆ geben die Ausbeute an TDA an. Sie bilden die Basis für die Trendkurve in Form eines Polynoms 3. Grades, welche mit Hilfe der Methode der kleinsten Quadrate ermittelt wurde (gepunktete Linie).

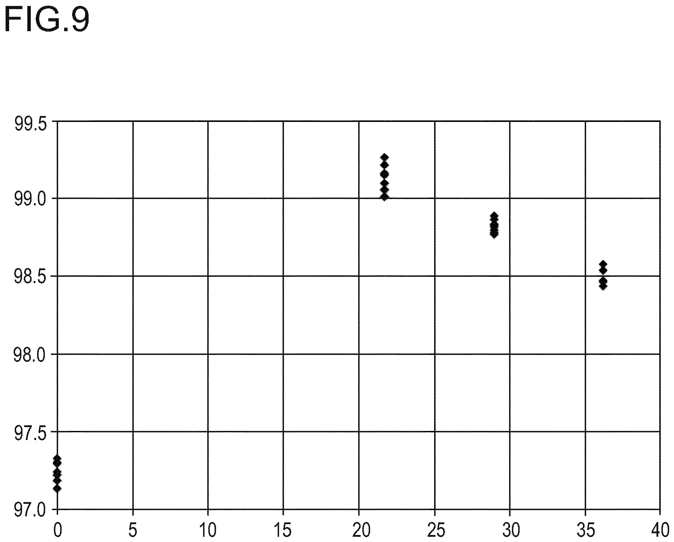

FIG. 9 gibt die TDA-Ausbeute bei Zudosierung steigender Kalium-Konzentrationen wieder.
   Die x-Achse des Diagramms beschreibt die Kalium-Konzentration im Reaktor in mmol/kg. Die y-Achse zeigt die TDA-Ausbeute in Flächen-% bezogen auf die Gesamtfläche aller Peaks bei der GC.
   Die Datenpunkte ◆ geben die Ausbeute an TDA an.

### Beispiele

Die folgenden Experimente werden in einem Laborreaktor-Aufbau durchgeführt. Dieser besteht aus einem Schlaufenreaktoraufbau, der überwiegend als Rohrreaktor ausgeführt ist, aber in welchem auch ein Rührkessel integriert ist. Im Rohrreaktorteil des Schlaufenreaktors werden Dinitrotoluol (DNT) und Wasserstoff in den Zirkulationsstrom bestehend aus Produkt und suspendierten Katalysator (3%Pt-1%Ni/C-Katalysator) eingedüst bzw. zudosiert und mit diesem vermischt. Kurz nach diesem Zugabepunkt ist ein zweiter Zugabepunkt installiert, über den Co-Dosierungen dem Zirkulationsstrom zugeführt werden können. Die Wasserstoffdosierung erfolgt druckgeregelt, so dass immer ausreichend Wasserstoff bei konstantem Druck verfügbar ist. Überschüssiges Produkt verlässt den Schlaufenreaktoraufbau über eine den Katalysator zurückhaltende Edelstahlfritte, die im Rührkessel installiert ist. Das filtrierte Produkt wird danach in einem Phasenscheider in eine Gas- und eine Flüssigphase getrennt und der Gasfluss auf 10Nl/h geregelt, um einen kontinuierlichen Gasaustrag zu gewährleisten und die Akkumulation von Inertgasen zu verhindern. Die Flüssigphase wird im Tagbetrieb regelmäßig entnommen und per Gaschromatographie (GC mit einer RTX-5 Amine Säule; Injektionstemperatur 260°C) analysiert.

### Beispiel 1

### Co-Dosiertest

Der Laborreaktor-Aufbau wird mit 3 g (Trockenmenge) Katalysator suspendiert in Wasser beladen und bei 185°C, 27 bar, einem Zirkulationsstrom von 36kg/h und einer DNT-Dosierrate von 100g/h betrieben. Daraus resultiert eine WHSV von 33,3kg(DNT)/kg(Kat)/h. Wie in FIG. 1 zu sehen ist, verbessert sich die TDA-Ausbeute graduell um ungefähr 1% während der Zudosierung von KHCO₃-Lösung zur Reaktion.

Dieser Effekt nimmt graduell sofort nach Beendigung der Zudosierung ab, weil das Salz mit dem Produkt aus dem Reaktorsystem gespült wird. Die gestrichelte Linie markiert eine Unterbrechung der DNT-Zufuhr über einen Zeitraum von fast 70 h, bei der der Zirkulationsstrom und alle anderen Bedingungen aufrechterhalten bleiben.

### Beispiel 2

### Co-Dosiertest

Der Laborreaktor-Aufbau wird wie zuvor betrieben, außer dass mit 5 g (Trockenmenge) Katalysator beladen wird, so dass die WHSV 20kg(DNT)/kg(Kat)/h beträgt.

Nach einer Einlaufperiode von 250 h und einer ausgeprägten Katalysatoralterung wird Kaliumbicarbonat hinzugegeben und die TDA-Ausbeute steigt graduell um 2% (s FIG. 2). Nach Abschaltung der Dosierung nimmt die TDA-Ausbeute graduell wieder ab. Anschließende Zugabe von Ammoniumbicarbonat ergibt keinen Effekt auf die TDA-Ausbeute, wohingegen die darauffolgende Zugabe von Kaliumhydroxid zu einer graduellen TDA-Ausbeutesteigerung führt. Daher kann die Steigerung der TDA-Ausbeute auf die Gegenwart von Kalium-Kationen zurückgeführt werden. Da die Reaktion im Produkt durchgeführt wird und dieses ohnehin von stark alkalischer Natur ist (>60% TDA in Wasser), kann ein Effekt des Kaliums auf den pH-Wert als Ursache ausgeschlossen werden.

In FIG. 3 sind die berechneten Konzentrationen der Kationen gemeinsam mit der TDA-Ausbeute dargestellt. Durch die kontinuierliche Zugabe und die Rückvermischung des Systems bauen sich die Salzkonzentrationen im Zirkulationsstrom langsam bzw. graduell auf und nehmen nach Beendigung der Zudosierung ebenso graduell ab. Gut zu erkennen ist, wie sich diese Zunahme und Abnahme in der TDA-Ausbeute widerspiegelt.

Sättigungskonzentrationen der Salze in mmol/kg errechnen sich mit Bezug auf die Konzentration von reinem DNT (5,49 mol/kg), d.h. 5,5 mmol/kg entsprechen 0,1 Gew.-% bezüglich des Edukts und beschreiben näherungsweise auch den Gewichtsanteil im Produkt (H₂-Aufnahme vernachlässigt).

### Beispiel 3

### Co-Dosiertest

Das folgende Experiment wird in einer Miniplant-Versuchsanlage durchgeführt. Diese besteht aus einem Schlaufenreaktoraufbau, der in einem Teil (5,6 L) über eine interne Zirkulationsströmung verfügt, die von einem Treibstrahl (Zirkulationsstrom bestehend aus Produkt und Katalysator) angetrieben wird, und in einem anderen Teil als Rohrreaktor ausgeführt ist (4,4 L). Der gesamte Aufbau wird mit Thermoöl thermostatisiert, um entstehende Wärme abzuführen. Nahe dem Treibstrahl wird DNT eingemischt und Wasserstoff wird druckgeregelt in den Gasraum oberhalb der internen Zirkulationsströmung dosiert. Gebildetes Produkt wird über eine den Katalysator zurückhaltende Membran entnommen, so dass der Flüssigstand im Reaktorteil mit der internen Zirkulationsströmung gleich bleibt. Dieser Austrag wird regelmäßig per GC analysiert. Eine feste Gasmenge wird oberhalb des Gasraums abgeführt, so dass keine unbegrenzte Ackumulation von gasförmigen Produkten oder Verunreinigungen erfolgt.

Der Reaktor wird mit 112 g (Trockenmenge) 3%Pt-1%Ni/C-Katalysator suspendiert in Wasser beladen und bei 185°C, 25 bar Überdruck, einem Zirkulationsstrom von 500 kg/h und einer DNT-Dosierrate von 2kg/h betrieben. Daraus resultiert eine WHSV von 17,9 kg(DNT)/kg(cat)/h.

Wie in FIG. 4 zu sehen ist, verbessert sich die TDA-Ausbeute um bis zu 0,7% durch pulsweise Dosierung von Kaliumbicarbonat in der höchsten Konzentration. Dieser Effekt nimmt schnell graduell ab, weil das Salz kontinuierlich mit dem Produkt ausgetragen wird. Die pulsweise Zugabe erfolgt durch Zugabe eines Volumens von 0,1 bzw. 0,2 L wässriger Salzlösung in den Reaktor. Dazu wird nach Abstellung der DNT-Zudosierung der Flüssigstand im Reaktor abgesenkt und das Volumen durch Öffnen eines Ventils mit Überdruck in den Reaktorkreislauf eingespeist. Im Anschluss wird die DNT-Zudosierung wieder aufgenommen und der Flüssigstand wieder auf den alten Wert geregelt.

Die Konzentrationen werden auf das gesamte Reaktorvolumen bezogen berechnet. Konzentrationen der Salze in mmol/kg errechnen sich mit Bezug auf die Konzentration von reinem DNT (5,49 mol/kg), d.h. 27,5 mmol/kg entsprechen 0,5 Gew.-% bezüglich des Edukts und beschreiben nahezu auch den Gewichtsanteil im Produkt (H₂-Aufnahme vernachlässigt). Erforderliche stationäre Konzentrationen bewegen sich im Bereich der Beispiele 1 und 2.

### Beispiel 4

Kalium-Co-Dosierungseffekt während Unterbrechung der DNT-Zugabe (=heißer Standby bzw. extrem lange Verweilzeit von bis zu mehreren Tagen)

Normalerweise führt die Unterbrechung der DNT-Zugabe bei Erhaltung der sonstigen Reaktionsbedingungen zu einer starken Abnahme der TDA-Ausbeute durch Bildung von Hochsiedern. Dieser Zustand entspricht einer Verlängerung der Verweilzeit, so dass der Katalysator nach Abreaktion des DNT hinsichtlich der Hydrierung von Nitrogruppen ungenutzt ist.

In FIG. 5 sind die bestimmten TDA-Ausbeuten gezeigt. Während der Dauer der Unterbrechung der DNT-Zugabe wird der TDA-Anteil des organischen Produktes (bzw. die TDA-Ausbeute) um 13% reduziert und steigt nach Wiederaufnahme der DNT-Zugabe graduell an, aber nicht auf die ursprüngliche TDA-Ausbeute, sondern auf einem Wert, der 1% niedriger ist.

FIG. 6 zeigt die TDA-Ausbeute vor und nach einer Unterbrechung der DNT-Zugabe in Gegenwart von Kaliumbicarbonat (Zudosierung vor Unterbrechung). Eine erkennbare Hochsiederbildung wird in diesem Fall nicht beobachtet und die TDA-Ausbeute ist ebenfalls stabil. Die nach Wiederaufnahme der DNT-Zudosierung abnehmende TDA-Ausbeute ist auf das Ausspülen des verbliebenen Kaliumbicarbonats zurückzuführen.

### Beispiel 5

Der Co-Dosiertest aus Beispiel 5 wird analog zu dem Co-Dosiertest aus Beispiel 2 durchgeführt. Der Co-Dosierungsstrom besteht nacheinander aus 4000 ppm Kaliumbicarbonat (1) (Endkonzentration 5,5mmol(K)/kg), 1600 ppm Strontiumnitrat (5) (Endkonzentration 1,0 mmol(Sr)/kg) und 10 000 ppm Strontiumnitrat (6) (Endkonzentration 6,5mmol(Sr)/kg) (jeweils wässrige Lösungen).

### Beispiel 6

Das folgende Experiment wird in einer Miniplant-Versuchsanlage durchgeführt. Diese besteht aus einem Schlaufenreaktoraufbau, der in einem Teil (5,6 L) über eine interne Zirkulationsströmung verfügt, die von einem Treibstrahl (Zirkulationsstrom bestehend aus Produkt und Katalysator) angetrieben wird, und in einem anderen Teil als Rohrreaktor ausgeführt ist (4,4 L). Der gesamte Aufbau wird mit Thermoöl thermostatisiert, um entstehende Wärme abzuführen. Nahe dem Treibstrahl wird DNT eingemischt und Wasserstoff wird druckgeregelt in den Gasraum oberhalb der internen Zirkulationsströmung dosiert. Gebildetes Produkt wird über eine den Katalysator zurückhaltende Membran entnommen, so dass der Flüssigstand im Reaktorteil mit der internen Zirkulationsströmung gleich bleibt. Eine feste Gasmenge wird oberhalb des Gasraums abgeführt, so dass keine unbegrenzte Akkumulation von gasförmigen Produkten oder Verunreinigungen erfolgt.

Der Reaktor wird mit 112 g (Trockenmenge) 3%Pt-1%Ni/C-Katalysator suspendiert in Wasser beladen und bei 185°C, 25 bar Überdruck, Zirkulationsstrom von 500 kg/h und einer DNT-Dosierrate von 4 kg/h betrieben. Daraus resultiert eine WHSV von 35,8 kg(DNT)/kg(cat)/h.

Die Zugabe von Kaliumcarbonat erfolgt durch Zuführung eines kontinuierlichen Volumenstroms einer 1% wässrigen Salzlösung in den Reaktor. Die Einleitung erfolgt durch ein Rohr, das am Reaktorkopf durchgeführt wird. Die Lösung tropft dabei in die interne Zirkulationsströmung des Reaktors am Rand des Einsteckrohres, d.h. entfernt von der DNT-Zugabestelle. Durch Variation des Volumenstroms der Salzlösung werden verschiedene Kalium-Konzentrationen eingestellt. Nach Einstellung eines stationären Zustandes werden mehrere Proben gezogen und per GC analysiert.

In FIG. 8 sind die resultierenden TDA-Ausbeuten gegen die resultierende stationäre Kaliumkonzentration aufgetragen. Es ist gut zu erkennen, dass es ein Optimum der Zugabe hinsichtlich der Verbesserung der TDA-Ausbeute gibt, das in diesem Fall bei ca. 10 mmol(K)/kg liegt. Das entspricht 0,19 mol-% K bezogen auf das zugeführte DNT.

### Beispiel 7

Das folgende Experiment wird analog zu Beispiel 6 durchgeführt mit dem Unterschied, dass die DNT-Dosierrate 2 kg/h (WHSV von 17,9 kg(DNT)/kg(cat)/h) beträgt und eine 4% ige wässrige Kaliumcarbonatlösung eingesetzt wird.

In FIG.9 sind analog zu FIG.8 die resultierenden TDA-Ausbeuten gegen die resultierende stationäre Kaliumkonzentration aufgetragen. Nachdem ein Optimum der Zugabe der Salzlösung hinsichtlich der Verbesserung der TDA-Ausbeute erreicht wurde, ist gut zu erkennen, dass eine weitere Steigerung der Kalium-Konzentration zu einer Verschlechterung der TDA Ausbeute führt. Das Optimum der Zugabe hinsichtlich der Verbesserung der TDA-Ausbeute bei dieser Fahrweise beträgt ca. 22 mmol(K)/kg. Das entspricht 0,36 mol-% K bezogen auf das zugeführte DNT. Eine weitere Steigerung auf ca. 28 mmol(K)/kg (entspricht ca. 0,5 mol-% K bezogen auf das zugeführte DNT) führt bereits zu einer deutlichen Verschlechterung der Selektivität um ca. 0,4 % in der TDA Ausbeute.

## Patentansprüche

1. Verfahren zur kontinuierlichen Hydrierung von Dinitrotoluol zu Toluylendiamin in einem Dinitrotoluol enthaltenden flüssigen Reaktionsgemisch in Gegenwart eines Trägerkatalysators, der als aktive Komponente ein Gemisch aus Nickel und Platin mit einem Atomverhältnis von Nickel zu Platin von 30:70 bis 70:30 sowie gegebenenfalls ein oder mehrere zusätzliche Metalle enthält, **dadurch gekennzeichnet, dass** die Hydrierung in Gegenwart mindestens eines Salzes ausgewählt aus der Gruppe bestehend aus den Salzen der Alkalimetalle, Erdalkalimetalle und der Seltenerdmetalle durchgeführt wird.

2. Verfahren Anspruch 1, **dadurch gekennzeichnet, dass** die aktive Komponente des Katalysators Chrom enthält.

3. Verfahren nach Anspruch 1 oder 2, **dadurch gekennzeichnet, dass** die aktive Komponente des Trägerkatalysators Nickel in Form von Nickelkristalliten mit einer bimodalen Nickelkristallitgrößenverteilung enthält und einen Nickel-Gehalt von 60 bis 80 Gew.-%, bezogen auf die Gesamtmasse des Katalysators, sowie einen Reduktionsgrad von mindestens 70% aufweist.

4. Verfahren nach Anspruch 1 oder 2, **dadurch gekennzeichnet, dass** der Katalysator basierend auf Nickel und Platin und mindestens einem zusätzlichen Metall
1 bis 5 Gew.-% Platin,
0,3 bis 1,5 Gew.-% Nickel,
0,05 bis 1,5 Gew.-% des mindestens einen zusätzlichen Metalls, sowie
94,65 bis 97,45 Gew.-% Trägermaterial,
jeweils bezogen auf das Gesamtgewicht des Katalysators, wobei die Summe 100 Gew.-% ergibt, enthält.

5. Verfahren nach einem der Ansprüche 1 bis 3, **dadurch gekennzeichnet, dass** das mindestens eine Salz im Katalysator enthalten ist.

6. Verfahren nach Anspruch 4 oder 5, **dadurch gekennzeichnet, dass** das zusätzliche Metall mindestens ein Metall, ausgewählt aus der Gruppe bestehend aus Kupfer, Kobalt, Eisen, Zink, Mangan und Chrom, ist.

7. Verfahren nach Anspruch 5, **dadurch gekennzeichnet, dass** das mindestens eine Salz im Katalysator insgesamt in einer Konzentration von 0,05 bis 20 Gew.-%, bezogen auf das Trockengewicht des Katalysators, vorliegt.

8. Verfahren nach einem der Ansprüche 1 bis 4, **dadurch gekennzeichnet, dass** das mindestens eine Salz im flüssigen Reaktionsgemisch enthalten ist.

9. Verfahren nach Anspruch 8, **dadurch gekennzeichnet, dass** das mindestens eine Salz im flüssigen Reaktionsgemisch insgesamt in einer Konzentration von 0,01 bis 1 mol-%, bezogen auf die zugeführte Menge an zu hydrierendem Dinitrotoluol, vorliegt.

10. Verfahren nach einem der Ansprüche 1 bis 9, **dadurch gekennzeichnet, dass** das mindestens eine Salz Kalium, Strontium, Natrium oder Gemische daraus enthält.

11. Verfahren nach einem der Ansprüche 1 bis 10, **dadurch gekennzeichnet, dass** das Salz ein Carbonat, Hydrogencarbonat, Hydroxid, Oxid, Nitrat oder Carboxylat oder ein Gemisch daraus ist.

12. Verfahren nach Anspruch 1, **dadurch gekennzeichnet, dass** das Reaktionsgemisch neben dem zu hydrierenden Dinitrotoluol mindestens einen Hochsieder, ausgewählt aus der Gruppe bestehend aus Dinitrokresolen, Trinitrokresolen und Nitrophenolen, enthält.

13. Verfahren nach einem der Ansprüche 1 bis 11, **dadurch gekennzeichnet, dass** das Reaktionsgemisch neben der zu hydrierenden Nitroverbindung keine Hochsieder, ausgewählt aus der Gruppe bestehend aus Dinitrokresolen, Trinitrokresolen und Nitrophenolen, enthält.

14. Verfahren nach einem der Ansprüche 1 bis 13, **dadurch gekennzeichnet, dass** das Reaktionsgemisch neben der zu hydrierenden Nitroverbindung mindestens eine Verbindung aus der Gruppe bestehend aus Salpetersäure, Schwefelsäure, Stickstoffoxide, Distickstoffmonoxid, Cyanwasserstoffsäure, Kohlenstoffmonoxid und Nitrobenzoesäure oder deren Abbauprodukte enthält.

15. Verfahren nach einem der Ansprüche 1 bis 14, **dadurch gekennzeichnet, dass** die Hydrierung bei einer Temperatur von 80 bis 250°C durchgeführt wird.

## Claims

1. A process for continuous hydrogenation of dinitrotoluene to tolylenediamine in a liquid reaction mixture comprising dinitrotoluene in the presence of a supported catalyst which comprises as the active component a mixture of nickel and platinum in an atom ratio of nickel to platinum of from 30:70 to 70:30 and optionally one or more additional metals, wherein the hydrogenation is performed in the presence of at least one salt selected from the group consisting of the salts of the alkali metals, alkaline earth metals and of the rare earth metals.

2. The process according to claim 1, wherein the active component of the catalyst comprises chromium.

3. The process according to claim 1 or 2, wherein the active component of the supported catalyst comprises nickel in the form of nickel crystallites having a bimodal nickel crystallite size distribution and has a nickel content of 60 to 80 wt% based on the total mass of the catalyst and a degree of reduction of at least 70%.

4. The process according to claim 1 or 2, wherein the catalyst based on nickel and platinum and at least one additional metal comprises
1 to 5 wt% of platinum,
0.3 to 1.5 wt% of nickel,
0.05 to 1.5 wt% of the at least one additional metal and
94.65 to 97.45 wt% of support material,
based in each case on the total weight of the catalyst, where the sum total is 100 wt%.

5. The process according to any of claims 1 to 3, wherein the at least one salt is comprised in the catalyst.

6. The process according to claim 4 or 5, wherein the additional metal is at least one metal selected from the group consisting of copper, cobalt, iron, zinc, manganese and chromium.

7. The process according to claim 5, wherein the at least one salt in the catalyst is present in a total concentration of 0.05 to 20 wt% based on the dry weight of the catalyst.

8. The process according to any of claims 1 to 4, wherein the at least one salt is comprised in the liquid reaction mixture.

9. The process according to claim 8, wherein the at least one salt in the liquid reaction mixture is present in a total concentration of 0.01 to 1 mol% based on the supplied amount of dinitrotoluene for hydrogenation.

10. The process according to any of claims 1 to 9, wherein the at least one salt comprises potassium, strontium, sodium or mixtures thereof.

11. The process according to any of claims 1 to 10, wherein the salt is a carbonate, hydrogencarbonate, hydroxide, oxide, nitrate or carboxylate or a mixture thereof.

12. The process according to claim 1, wherein in addition to the dinitrotoluene for hydrogenation the reaction mixture comprises at least one high boiler selected from the group consisting of dinitrocresols, trinitrocresols and nitrophenols.

13. The process according to any of claims 1 to 11, wherein in addition to the nitro compound for hydrogenation the reaction mixture comprises no high boilers selected from the group consisting of dinitrocresols, trinitrocresols and nitrophenols.

14. The process according to any of claims 1 to 13, wherein in addition to the nitro compound for hydrogenation the reaction mixture comprises at least one compound from the group consisting of nitric acid, sulfuric acid, nitrogen oxides, dinitrogen monoxide, hydrocyanic acid, carbon monoxide and nitrobenzoic acid or degradation products thereof.

15. The process according to any of claims 1 to 14, wherein the hydrogenation is performed at a temperature of 80°C to 250°C.

## Revendications

1. Procédé pour l'hydrogénation continue de dinitrotoluène en toluylène-diamine dans un mélange réactionnel liquide contenant du dinitrotoluène en présence d'un catalyseur supporté, qui contient en tant que composant actif un mélange de nickel et de platine ayant un rapport atomique entre le nickel et le platine de 30:70 à 70:30, ainsi qu'éventuellement un ou plusieurs métaux supplémentaires, **caractérisé en ce que** l'hydrogénation est réalisée en présence d'au moins un sel choisi dans le groupe constitué par les sels des métaux alcalins, des métaux alcalino-terreux et des métaux de terres rares.

2. Procédé selon la revendication 1, **caractérisé en ce que** le composant actif du catalyseur contient du chrome.

3. Procédé selon la revendication 1 ou 2, **caractérisé en ce que** le composant actif du catalyseur supporté contient du nickel sous la forme de cristallites de nickel ayant une distribution des tailles de cristallites de nickel bimodale et présente une teneur en nickel de 60 à 80 % en poids, par rapport à la masse totale du catalyseur, ainsi qu'un degré de réduction d'au moins 70 %.

4. Procédé selon la revendication 1 ou 2, **caractérisé en ce que** le catalyseur à base de nickel et de platine et d'au moins un métal supplémentaire contient
1 à 5 % en poids de platine,
0,3 à 1,5 % en poids de nickel,
0,05 à 1,5 % en poids dudit au moins un métal supplémentaire, ainsi que
94,65 à 97,45 % en poids de matériau support,
à chaque fois par rapport au poids total du catalyseur, la somme étant de 100 % en poids.

5. Procédé selon l'une quelconque des revendications 1 à 3, **caractérisé en ce que** ledit au moins un sel est contenu dans le catalyseur.

6. Procédé selon la revendication 4 ou 5, **caractérisé en ce que** le métal supplémentaire est au moins un métal choisi dans le groupe constitué par le cuivre, le cobalt, le fer, le zinc, le manganèse et le chrome.

7. Procédé selon la revendication 5, **caractérisé en ce que** ledit au moins un sel est présent dans le catalyseur au total en une concentration de 0,05 à 20 % en poids, par rapport au poids sec du catalyseur.

8. Procédé selon l'une quelconque des revendications 1 à 4, **caractérisé en ce que** ledit au moins un sel est contenu dans le mélange réactionnel liquide.

9. Procédé selon la revendication 8, **caractérisé en ce que** ledit au moins un sel est présent dans le mélange réactionnel liquide au total en une concentration de 0,01 à 1 % en moles, par rapport à la quantité ajoutée au dinitrotoluène à hydrogéner.

10. Procédé selon l'une quelconque des revendications 1 à 9, **caractérisé en ce que** ledit au moins un sel contient du potassium, du strontium, du sodium ou des mélanges de ceux-ci.

11. Procédé selon l'une quelconque des revendications 1 à 10, **caractérisé en ce que** le sel est un carbonate, un hydrogénocarbonate, un hydroxyde, un oxyde, un nitrate ou un carboxylate ou un mélange de ceux-ci.

12. Procédé selon la revendication 1, **caractérisé en ce que** le mélange réactionnel contient en plus du dinitrotoluène à hydrogéner au moins un composant de point d'ébullition élevé, choisi dans le groupe constitué par les dinitrocrésols, les trinitrocrésols et les nitrophénols.

13. Procédé selon l'une quelconque des revendications 1 à 11, **caractérisé en ce que** le mélange réactionnel ne contient pas de composant de point d'ébullition élevé, choisi dans le groupe constitué par les dinitrocrésols, les trinitrocrésols et les nitrophénols, en plus du composé nitro à hydrogéner.

14. Procédé selon l'une quelconque des revendications 1 à 13, **caractérisé en ce que** le mélange réactionnel contient en plus du composé nitro à hydrogéner au moins un composé du groupe constitué par l'acide nitrique, l'acide sulfurique, les oxydes d'azote, le monoxyde de diazote, l'acide cyanhydrique, le monoxyde de carbone et l'acide nitrobenzoïque ou leurs produits de décomposition.

15. Procédé selon l'une quelconque des revendications 1 à 14, **caractérisé en ce que** l'hydrogénation est réalisée à une température de 80 à 250 °C.
